# EUROPÄISCHE PATENTSCHRIFT

(11) **EP 2 994 153 B1**
(45) Veröffentlichungstag und Bekanntmachung des Hinweises auf die Patenterteilung: **15.08.2018**
(21) Anmeldenummer: 14720485.3
(22) Anmeldetag: 16.04.2014
(51) Int. Cl.: A61K 51/08, C07K 7/08

(54) **PEPTIDE UND PEPTID-WIRKSTOFF-KONJUGATE FÜR RENALES TARGETING**
PEPTIDES AND PEPTIDE-ACTIVE INGREDIENT-CONJUGATE FOR RENAL DRUG-TARGETING
PEPTIDES ET CONJUGUÉS PEPTIDE-PRINCIPE ACTIF POUR CIBLAGE RÉNAL

(30) Priorität: 07.05.2013 EP 13002432
(43) Veröffentlichungstag der Anmeldung: 16.03.2016
(73) Patentinhaber: Merck Patent GmbH, 64293 Darmstadt (DE)
(72) Erfinder: KUEBELBECK, Armin, 64625 Bensheim (DE); LARBIG, Gregor, 63571 Gelnhausen (DE); ARNOLD, Stefan, 64839 Muenster (DE); MIER, Walter, 64673 Bensheim (DE)
(86) Internationale Anmeldenummer: PCT/EP2014/001026
(87) Internationale Veröffentlichungsnummer: WO 2014/180534

(56) Entgegenhaltungen:
- WO-A1-98/11126
- WO-A1-99/46283
- WO-A1-2007/022774
- WO-A2-01/92469
- WO-A2-2008/089738
- CAIRO C W ET AL: "AFFINITY-BASED INHIBITION OF BETA-AMYLOID TOXICITY", BIOCHEMISTRY, AMERICAN CHEMICAL SOCIETY, US, Bd. 41, Nr. 27, 12. September 2002 (2002-09-12), Seiten 8620-8629, XP001184169, ISSN: 0006-2960, DOI: 10.1021/BI0156254
- LYU P C ET AL: "Energetic contribution of solvent-exposed ion pairs to alpha-helix structure", JOURNAL OF MOLECULAR BIOLOGY, ACADEMIC PRESS, UNITED KINGDOM, Bd. 223, Nr. 1, 5. Januar 1992 (1992-01-05), Seiten 343-350, XP024018331, ISSN: 0022-2836, DOI: 10.1016/0022-2836(92)90735-3 [gefunden am 1992-01-05]
- DOLMAN M E M ET AL: "Drug targeting to the kidney: Advances in the active targeting of therapeutics to proximal tubular cells", ADVANCED DRUG DELIVERY REVIEWS, ELSEVIER, AMSTERDAM, NL, Bd. 62, Nr. 14, 30. November 2010 (2010-11-30), Seiten 1344-1357, XP027511625, ISSN: 0169-409X, DOI: 10.1016/J.ADDR.2010.07.011 [gefunden am 2010-08-16]

## Beschreibung

Die vorliegende Erfindung betrifft ein Peptid welches zu mehr als 50% aus Sequenzabschnitten ausgewählt aus der Gruppe umfassend -(KKEEE)-, -(RREEE)-, -(KKEE)-, -(KKKEEE)- und -(KKKEE)- besteht, sowie ein Konjugat enthaltend das Peptid und mindestens einen kovalent gebundenen Wirkstoff und ein Verfahren zur Herstellung des Konjugats. Weiterhin betrifft die vorliegende Erfindung das Peptid und das Konjugat zur Verwendung zum Targeting der Niere, sowie ein Arzneimittel enthaltend das Peptid oder Konjugat.

Die Niere ist insbesondere für den Transport und die Ausscheidung verschiedener Substanzen und bei der Produktion von Hormonen von Bedeutung. Eine Funktion der Nieren ist die Ausscheidung von Endprodukten des Stoffwechsels, den sogenannten harnpflichtigen Substanzen, und Giftstoffen aus dem Körper durch Bildung des Harns, welcher schließlich über die Harnwege aus dem Körper ausgeschieden wird. Die Niere bilanziert den Wasserhaushalt und dient damit der langfristigen Blutdruckeinstellung. Sie reguliert durch die Kontrolle der Zusammensetzung des Harns den Elektrolythaushalt und den Säure-Basen-Haushalt. Weiterhin ist die Niere ein bedeutendes Organ für den Zwischenstoffwechsel des Körpers (sie betreibt Gluconeogenese). Die Niere produziert Hormone, wie beispielsweise Erythropoetin, für die Blutbildung und ist der Abbauort von Peptidhormonen. Aber auch viele Funktionen der Niere selbst werden durch Hormone gesteuert. Derzeit leiden ca. 280 Millionen Menschen an chronischen Nierenerkrankungen. Es wurden bereits viele diagnostische und therapeutische Methoden entwickelt. Beispielsweise werden zur Behandlung der Niere bzw. zur Beeinflussung der Nierenfunktion Immunsuppressiva, Zytostatika, Immuntherapeutika, Antiphlogistika, Antibiotika, Virostatika, Antihypertensiva, Urikosurika, oder Diuretika eingesetzt. Der Einsatz bzw. die Dosierung von Medikamenten zur Behandlung von Nierenerkrankungen ist jedoch häufig eingeschränkt durch Nebenwirkungen der Medikamente. Daher ist es besonders wichtig, dass die Medikamente so gezielt wie möglich in die Niere gelangen.

Genauso ist auch die Darstellung der Niere in bildgebenden Verfahren von großer Bedeutung.

Mit Hilfe der etablierten nuklearmedizinischen und radiologischen Verfahren wie Computer-Tomographie (CT), SPECT (Single-Photon-Emissionscomputertomographie), PET (Positronen-Emissions-Tomographie), Ultraschall und MRT (Magnetresonanztomographie) können neben morphologischen Strukturen durch die sogenannte molekulare Bildgebung enzymatische Prozesse, Stoffwechselvorgänge, die Expression bestimmter Gene und molekulare Reaktionen dargestellt werden. Die oben genannten Bildgebungsmodalitäten können eventuell weiter durch computertomographische und optische Bildgebungsverfahren (Near-Infrared-Imaging, Fluoreszenztomographie) ergänzt werden. Der Schwerpunkt des "Molecular Imaging" liegt derzeit noch in der Diagnostik von Krebserkrankungen, neurologischen Fragestellungen und der Kontrolle von Gentherapien, wird jedoch in der Zukunft auf alle Bereiche, in denen zelluläre Veränderungen möglichst frühzeitig entdeckt werden müssen, erweitert werden.
Als Signalquelle wird für die bildgebenden Verfahren in der Regel ein "Signalmolekül" mit einem "Trägermolekül" gekoppelt. Das "Trägermolekül" sorgt für das möglichst gezielte Targeting, indem es z.B. spezifisch an die Zielzellen bindet oder in diesen getrappt wird. Beispielsweise kann es sich bei dem Trägermolekül um den Liganden eines Rezeptors oder um das Substrat eines Enzyms handeln. Das "Signalmolekül" kann mittels einer oder mehrerer Bildgebungstechniken sichtbar gemacht werden. Beispiele für Signalmoleküle sind z.B. Komplexbildner oder Chelatoren, deren Metallionen über Bildgebungstechniken detektiert werden können. Die Verbindung bzw. das Konjugat aus Signalmolekül und Trägermolekül wird "Diagnostikum" genannt.

Für Untersuchungen der Niere wird insbesondere die Nierenszintigraphie genutzt. Diese ist ein nuklearmedizinisches Untersuchungsverfahren, welches die Beurteilung der Nierenfunktion unter statischen und dynamischen Gesichtspunkten erlaubt. Beurteilt werden dabei die Blutversorgung, Funktion und Exkretion jeder einzelnen Niere. Es ist ein etabliertes Verfahren zur Erkennung von Parenchymnarben, insbesondere bei Kindern und dient weiter zur Beurteilung der regionalen und seitengetrennten Nierenfunktion.
Bei der statischen Nierenszintigraphie wird unter Verwendung des Radionuklids ^{99m}Tc das funktionsfähige Nierengewebe dargestellt. Das Technetium ist dabei beispielsweise an 2,3-Dimercaptobernsteinsäure (DMSA) komplex gebunden. Die statische Nierenszintigraphie eignet sich daher vor allem zur Darstellung von Nieren mit Anomalien (Dystrophie, Hufeisenniere etc.) oder Zustand nach Entzündung.
Die dynamische Nierenszintigraphie untersucht demgegenüber die Nierenfunktion. So können die glomeruläre Filtrationsrate, der renale Blutfluss (RBF="renal blood flow") und die tubuläre Sekretion mit der Fragestellung nach der Nierenfunktion und ihrer Clearance untersucht werden.

Als Radiopharmaka kommen gegenwärtig die folgenden Substanzen zum Einsatz:
- ^{99m}Tc-MAG3 Mercaptoacetyltriglycin
- ^{99m}Tc-DMSA 2,3-Dimercaptobersteinsäure
- ^{99m}Tc-DTPA Diethylentriaminpentaessigsäure
- ¹²³I-OIH Hippuran (Orthoiodhippursäure)

Daher wäre es zum Beispiel sowohl für die Darstellung der Niere in bildgebenden Verfahren als auch für therapeutische Zwecke wünschenswert, wenn das Targeting der Niere verbessert werden könnte.

Im Stand der Technik sind bereits Substanzen bekannt, die sich zum Targeting der Niere, d.h. zum zielgesteuerten Transport in die Niere, eignen.

Beispielsweise ist bekannt, dass kleinere körpereigene Proteine, wie Lysozym (14,3 kDa), das Glomerulum der Nieren passieren können und sich als Transporter für die Adressierung der Nieren mit Wirkstoffen eignen (Franssen et al.: J. Med. Chem. 35, 7, 1992, 1246-1259; Zhang et al.: Biomaterials 30, 2009, S. 1372-1381). Nachteilig ist jedoch, dass Lysozym ein vergleichsweise großes Molekül ist, wodurch das Verhältnis Transporter zu Wirkstoff ungünstig ist. Außerdem erfolgt die Anbindung eines Wirkstoffes unspezifisch an einer der vielen vorhandenen reaktiven Seitengruppen. Dadurch entstehen chemisch undefinierte WirkstoffTransporter-Gemische. Darüber hinaus können Proteine wie Lysozym ein immunogenes Potential besitzen. Die Nachteile der hochmolekularen Proteine entfallen bei der Verwendung niedermolekularer Peptide.

In der aktuellen Literatur werden weiterhin verschiedene Peptide mit ca. 5 bis 20 Aminosäuren beschrieben, die selektiv von den Nieren aufgenommen werden. Beispielsweise handelt es sich hierbei um APASLYN und HITSLLS (Denby et al.: Molecular Therapy 15, 9, 2007, 1647-1654) oder ANTPCGPYTHDCPVKR (Kumar und Deutscher: The Journal of Nuclear Medicine 49, 5, 2008, 796-803; Geng et al.: Bioconjugate Chemistry 23, 2012, 1200-1210).

WO 2011/009539 A1 offenbart Wirkstoff-ε-Polylysin-Konjugate und deren hochselektive Anreicherung in der Niere. Die Verknüpfung der Lysin-Einheiten im Polymer erfolgt über ihre ε-Aminogruppen. Diese Verbindungen weisen eine sehr hohe Verweildauer in der Niere auf und werden dementsprechend relativ langsam abgebaut.

WO 2007/022774 A1) beschreibt die Verwendung von bestimmten Peptiden zum Schutz der Nieren vor Cisplatin-induziertem Nierenversagen.

WO 98/11126 beschreibt Peptid-Produrgs mit alpha-Hydroxysäure Linkern.

WO 99/46283 offenbart pharmakologisch aktive Peptidkonjugate.

WO 2008/089738 A2 beschreibt Eisenoxid-bindende Peptide, welche in medizinischen Anwendungen eingesetzt werden.

Cairo et al. (Cairo et al. 2002, Biochemistry 41: 8620-8629) beschreibt die Affinität von bestimmten Peptiden gegenüber beta-Amyloid.

Lyu et al. (Lyu et al. 1992, J. Mol. Biol. 223: 343-350) offenbart ein Peptid enthaltend die Sequenz (KKEE)₃ in Zusammenhang mit Studien zur Bestimmung der Rolle von Aminosäurenseitenkettenwechselwirkungen bei der Ausbildung der Sekundärstruktur in Proteinen. Es wird nicht offenbart, dass sich diese Peptide zum Targeting der Niere eignen bzw. zum Schutz der Niere verwendet werden können.

Es bestand daher weiterhin bedarf nach neuen Substanzen oder Trägermolekülen (engl. "Carrier"), die eine möglichst hohe Affinität und Selektivität für die Niere aufweisen. Dabei war es wünschenswert Substanzen zu finden, die selbst oder auch als Trägermoleküle mit ihren konjugierten Wirkstoffen innerhalb eines angemessenen Zeitraums in den Zielzellen der Niere biochemisch abbaubar sind.
Aufgabe der vorliegenden Erfindung war daher die Bereitstellung von Substanzen, die sich zum Targeting der Niere eignen, insbesondere auch als Trägermolekül für ein Therapeutikum oder ein Diagnostikum.
Es wurde überraschend gefunden, dass Peptide spezieller Aminosäuresequenzen und deren Wirkstoff-Konjugate eine sehr hohe Selektivität für die Niere aufweisen und auch rasch wieder abgebaut werden können. Die Peptide können als Konjugate mit Signalmolekülen wie z.B. Radioisotopen und/oder Wirkstoffen für die diagnostische und/oder therapeutische Behandlung der Niere eingesetzt werden.
Gegenstand der vorliegenden Erfindung ist daher ein Peptid oder Konjugat zur Verwendung zum Targeting oder Schutz der Niere, wie in Anspruch 1 und 2 beschrieben.
Gegenstand der vorliegenden Beschreibung ist ein Peptid, welches zu mehr als 50% (bezogen auf die Anzahl der Aminosäureeinheiten) aus Sequenzabschnitten der Formel (1)

-(Aₙ-Bₘ-Cₒ)- (1)

besteht, wobei
- A: für eine Aminosäure mit saurer Seitengruppe steht,
- B: für eine Aminogruppe mit basischer Seitengruppe steht,
- C: für eine beliebige Aminosäure steht,
- n, m: unabhängig voneinander für eine ganze Zahl von 1 bis 10 stehen, mit n:m = 1:3 bis 3:1,
- o: für eine ganze Zahl zwischen 0 und 10 steht,
und wobei
- das Peptid insgesamt eine Kettenlänge von 5 bis 100 Aminosäureeinheiten umfasst und
- das Peptid zu mindestens 50 % (bezogen auf die Anzahl der Aminosäureeinheiten) aus den Aminosäuren A und B besteht.

Unter einem Peptid versteht man erfindungsgemäß eine Verbindung, die aus einer Verknüpfung zweier oder mehrerer Aminosäuren über Amidbindungen entstanden ist. Dabei sind die einzelnen Aminosäuren in einer definierten Reihenfolge (Sequenz) zu einer Kette verbunden. Erfindungsgemäß sind Aminosäuren Verbindungen, die mindestens eine Aminogruppe und mindestens eine Carboxylgruppe tragen. Beispiele sind natürliche, proteinogene Aminosäuren oder nicht-proteinogene in Organismen vorkommende oder synthetisch hergestellte Aminosäuren.

Die Aminosäureeinheiten können im Peptid in der D- oder L-Form vorliegen.

Das Peptid umfasst 5 bis 100 Aminosäuren. In einer bevorzugten Form weist das Peptid eine Kettenlänge von 5 bis 40 Aminosäureeinheiten auf, besonders bevorzugt eine Kettenlänge von 10 bis 30 Aminosäureeinheiten.

Das Peptid besteht zu mehr als 50% (bezogen auf die Anzahl der Aminosäureeinheiten) aus Sequenzabschnitten der Formel (1)

-(Aₙ-Bₘ-Cₒ)- (1).

Bevorzugt besteht es zu mehr als 70 % aus Sequenzabschnitten der Formel (1), besonders bevorzugt zu mehr als 90%.

In Formel (1) steht A für eine Aminosäure mit saurer Seitengruppe. Hierbei kann es sich beispielsweise um Asparaginsäure, Glutaminsäure, Argininosuccinat und/oder Cysteinsäure handeln. Bevorzugt handelt es sich um Aminosäuren mit Carboxyl-Funktion, das heißt um Glutaminsäure und/oder Asparaginsäure, besonders bevorzugt um Glutaminsäure. Innerhalb eines Peptids kann A für verschiedene Aminosäuren mit sauren Seitengruppen stehen, das heißt es können beispielsweise gleichzeitig sowohl Glutaminsäure- als auch Asparaginsäure, Argininosuccinat und/oder Cysteinsäure -Reste im Peptid vorhanden sein.
In einer alternativen Ausführungsform sind die Aminosäuren mit sauren Seitengruppen A innerhalb eines Sequenzabschnitts des Peptids identisch; in diesem Fall stehen beispielsweise alle Aminosäuren A der Formel (1) in einem Sequenzabschnitt des Peptids für Asparaginsäure, Glutaminsäure, Argininosuccinat oder Cysteinsäure, in einem weiteren Sequenzabschnitt des Peptids unabhängig vom zuvor genannten Sequenzabschnitt für Asparaginsäure, Glutaminsäure oder Cysteinsäure.
In einer weiteren alternativen Ausführungsform sind die Aminosäuren mit sauren Seitengruppen A innerhalb des Peptids identisch; in diesem Fall stehen alle Aminosäuren A des Peptids beispielsweise für Asparaginsäure, Glutaminsäure, Argininosuccinat oder Cysteinsäure.
In einer bevorzugten Ausführungsform stehen alle Aminosäuren A innerhalb des Peptids für Glutaminsäure.

n definiert in Formel (1) die Zahl der Aminosäureeinheiten A. Dabei steht n für eine ganze Zahl von 1 bis 10. Bevorzugt steht n für eine ganze Zahl von 1 bis 5, besonders bevorzugt für 2 oder 3.

In Formel (1) steht B für eine Aminosäure mit basischer Seitengruppe. Hierbei kann es sich beispielsweise um Lysin, Arginin, Histidin und/ oder Ornithin handeln. Bevorzugt handelt es sich um Lysin.
Innerhalb eines Peptids kann B für verschiedene Aminosäuren mit basischen Seitengruppen stehen, das heißt es können beispielsweise gleichzeitig sowohl Lysin-, Arginin-, Histidin- und/ oder Ornithinreste im Peptid vorhanden sein.

In einer alternativen Ausführungsform sind die Aminosäuren mit basischen Seitengruppen B innerhalb eines Sequenzabschnitts des Peptids identisch; in diesem Fall stehen beispielsweise alle Aminosäuren B der Formel (1) in einem Sequenzabschnitt des Peptids für Lysin, Arginin, Histidin oder Ornithin, in einem weiteren Sequenzabschnitt des Peptids unabhängig vom zuvor genannten Sequenzabschnitt für Lysin, Arginin, Histidin oder Ornithin.
In einer weiteren alternativen Ausführungsform sind die Aminosäuren mit basischen Seitengruppen B innerhalb des Peptids identisch; in diesem Fall stehen alle Aminosäuren B des Peptids beispielsweise für Lysin, Arginin, Histidin oder Ornithin.
In einer bevorzugten Ausführungsform stehen alle Aminosäuren B innerhalb des Peptids für Lysin.

m definiert in Formel (1) die Zahl der Aminosäureeinheiten B. Dabei steht m für eine ganze Zahl von 1 bis 10. Bevorzugt steht m für eine ganze Zahl von 1 bis 5, besonders bevorzugt für 2 oder 3.

In Formel (1) steht C für eine beliebige Aminosäure. Hierbei kann es sich beispielsweise handeln um Alanin, Arginin, Asparagin, , Cystein, Glutamin, Glycin, Histidin, Isoleucin, Leucin, Lysin, Methionin, Phenylalanin, Prolin, Serin, Threonin, Tryptophan, Tyrosin, Valin und/oder Citrullin.
Bevorzugt handelt es sich um proteinogene Aminosäuren, die in natürlicher Weise verknüpft sind. Dies gewährleistet den Abbau des Peptids in den proximalen Tubuluszellen der Nieren zu toxikologisch völlig unbedenklichen Metaboliten.
Innerhalb eines Peptids kann C für verschiedene Aminosäuren stehen.

o definiert in Formel (1) die Zahl der Aminosäureeinheiten C. Dabei steht o für eine ganze Zahl von 0 bis 10. Bevorzugt steht o für 0, 1 oder 2, besonders bevorzugt für 0 oder 1. In einer ganz besonders bevorzugten Ausführungsform steht o für 0, das heißt in diesem Fall ist keine Aminosäureeinheit C im Peptid enthalten.

In einer bevorzugten Ausführungsform stehen n und m unabhängig voneinander für 2 oder 3.

Das Verhältnis von n:m in Formel (1) ist bei 1:3 bis 3:1. Beispielhafte Ausführungsformen der Sequenzabschnitte der Formel (1) sind: -(A₁-B₃-Cₒ)-, -(A₁-B₂-Cₒ)-, -(A₁-B₁-Cₒ)-, -(A₂-B₆-Cₒ)-, -(A₂-B₅-Cₒ)-, -(A₂-B₄-Cₒ)-, -(A₂-B₃-Cₒ)-, -(A₂-B₂-Cₒ)-, -(A₂-B₁-Cₒ)-, -(A₃-B₉-Cₒ)-, -(A₃-B₈-Cₒ)-, -(A₃-B₇-Cₒ)-, -(A₃-B₆-Cₒ)-, -(A₃-B₅-Cₒ)-, -(A₃-B₄-Cₒ)-, -(A₃-B₃-Cₒ)-, -(A₃-B₂-Cₒ)-,-(A₃-B₁-Cₒ)-, -(A₄-B₁₀-Cₒ)-, -(A₄-B₉-Cₒ)-, -(A₄-B₃-Cₒ)-, -(A₄-B₇-Cₒ)-, -(A₄-B₆-Cₒ)-, -(A₄-B₅-Cₒ)-, -(A₄-B₄-Cₒ)-, -(A₄-B₃-Cₒ)-, -(A₄-B₂-Cₒ)-, -(A₅-B₁₀-Cₒ)-, -(A₅-B₉-Cₒ)-, -(A₅-B₈-Cₒ)-, -(A₅-B₇-Cₒ)-, -(A₅-B₆-Cₒ)-, -(A₅-B₅-Cₒ)-, -(A₅-B₄-Cₒ)-,-(A₅-B₃-Cₒ)-, -(A₅-B₂-Cₒ)-, -(A₆-B₁₀-Cₒ)-, -(A₆-B₉Cₒ)-, -(A₆-B₈-Cₒ)-, -(A₆-B₇-Cₒ)-, -(A₆-B₆-Cₒ)-, -(A₆-B₅-Cₒ)-, -(A₆-B₅-Cₒ)-, -(A₆-B₃-Cₒ)-, -(A₆-B₂-Cₒ)-, -(A₇-B₁₀-Cₒ)-, -(A₇-B₉-Cₒ)-, -(A₇-B₈-Cₒ)-, -(A₇-B₇-Cₒ)-, -(A₇-B₆-Cₒ)-, -(A₇-B₅-Cₒ)-,-(A₇-B₄-Cₒ)-, -(A₇-B₃-Cₒ)-, -(A₈-B₁₀-Cₒ)-, -(A₈-B₉-Cₒ)-, -(A₈-B₈-Cₒ)-, -(A₈-B₇-Cₒ)-, -(A₈-B₅-Cₒ)-, -(A₈-B₅-Cₒ)-, -(A₈-B₄-Cₒ)-, -(A₈-B₃-Cₒ)-, -(A₉-B₁₀-Cₒ)-, -(A₉-B₉-Cₒ)-, -(A₉-B₈-Cₒ)-, -(A₉-B₇-Cₒ)-, -(A₉-B₆-Cₒ)-, -(A₉-B₅-Cₒ)-, -(A₉-B₄-Cₒ)-,-(A₉-B₃-Cₒ)-, -(A₁₀-B₁₀-Cₒ)-, -(A₁₀-B₉-Cₒ)-, -(A₁₀-B₈-Cₒ)-, -(A₁₀-B₇-Cₒ)-, -(A₁₀-B₆-Cₒ)-, -(A₁₀-B₅-Cₒ)- oder -(A₁₀-B₄-Cₒ)-, wobei A, B, C und o wie zuvor beschrieben definiert sind.

Die Sequenz der Formel (1) kann beispielsweise stehen für eine Sequenz ausgewählt aus
-(EKKK)-, -(EKK)-, -(EK)-, -(EEKKKKK)-, -(EEKKKK)-, -(EEKKK)-, -(EEKK)-, -(EEK)-, -(EEEKKKKK)-, -(EEEKKKK)-, -(EEEKKK)-, -(EEEKK)-, -(EEEK)-, -(EEEEKKKKK)-, -(EEEEKKKK)-, -(EEEEKKK)-, -(EEEEKK)-,-(EEEEEKKKKK)-, -(EEEEEKKKK)-, -(EEEEEKKK)-, -(EEEEEEKK)-, -(DKKK)-, -(DKK)-, -(DK)-, -(DDKKKKK)-, -(DDKKKK)-, -(DDKKK)-, - (DDKK)-, -(DDK)-, -(DDDKKKKK)-, -(DDDKKKK)-, -(DDDKKK)-, -(DDDKK)-, -(DDDK)-, -(DDDDKKKKK)-, -(DDDDKKKK)-, -(DDDDKKK)-, -(DDDDKK)-, -(DDDDDKKKKK)-, -(DDDDDKKKK)-, -(DDDDDKKK)-, -(DDDDDDKK)-, -(ERRR)-, -(ERR)-, -(ER)-, -(EERRRRR)-, -(EERRRR)-, -(EERRR)-, - (EERR)-, -(EER)-, -(EEERRRRR)-, -(EEERRRR)-, -(EEERRR)-, -(EEERR)-, -(EEER)-, -(EEEERRRRR)-, -(EEEERRRR)-, -(EEEERRR)-, -(EEEERR)-, -(EEEEERRRRR)-, -(EEEEERRRR)-, -(EEEEERRR)-, -(EEEEEERR)-, -(EKRK)-, -(ERK)-, -(EDKKRRK)-, -(EDKKKK)-, -(ECKKH)-, -(EDKK)-, - (DEEKKKHK)-, -(EDDKKKK)-, -(EDERRR)-, -(DCEKH)-, -(DEEK)-, - (DEDERKRKR)-, -(DEEDKKKH)-, -(EDCEKRH)-, -(EDDEKK)-, - (EEEEEKKRRK)-, -(EEEEDKKRK)-, -(EDDEEKKR)-, -(DDEEEEKK)-, worin jeweils die Einbuchstaben-Codes der Aminosäuren benutzt werden: E (Glutaminsäure), D (Asparaginsäure), C (Cystein), K (Lysin), R (Arginin), H (Histidin).

Erfindungsgemäß steht die Sequenz der Formel (1) für eine Sequenz ausgewählt aus der Gruppe umfassend -(KKEEE)-, -(RREEE)-, -(KKEE)-, - (KKKEEE)- und -(KKKEE)-.
Besonders bevorzugt steht die Sequenz der Formel (1) für die Sequenz -(KKEEE)-:

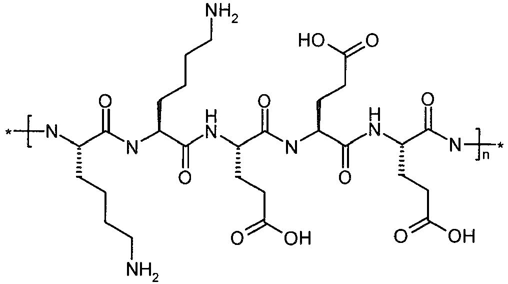

Erfindungsgemäß besteht das Peptid zu mindestens 80 % (bezogen auf die Anzahl der Aminosäureeinheiten) aus den Aminosäuren K und E bzw. R und E.

Insgesamt kann der Sequenzabschnitt der Formel (1) im Peptid 1 bis 50 mal enthalten sein, bevorzugt 1 bis 30 mal, besonders bevorzugt 1 bis 10 mal, insbesondere bevorzugt 2 bis 5 mal.

In einer möglichen Ausführungsform enthält das Peptid mehrere direkt aufeinanderfolgende Sequenzabschnitte der Formel (1). Bevorzugt enthält das Peptid 3 bis 5 aufeinanderfolgende Sequenzabschnitte der Formel (1). Beispielsweise kann das Peptid aus 3 bis 5 aufeinanderfolgenden Sequenzabschnitten der Formel (1) sowie einer oder mehreren weiteren Aminosäuren am C- und/ oder N-Terminus bestehen. Dies ist in Formel (2) veranschaulicht:

Xₚ(AₙBₘCₒ)ₓY_{q} (2)

worin A, B, C, n, m und o wie zuvor definiert sind,
x für 3, 4, oder 5 steht,
X und Y unabhängig voneinander für eine beliebige Aminosäure, bevorzugt für A, stehen und
p und q unabhängig voneinander für eine ganze Zahl zwischen 0 und 3 stehen, bevorzugt für 0 oder 1.

Beispiele für mögliche Peptide sind Peptide ausgewählt aus der Gruppe umfassend (RREEE)₃R, (KKEE)₅K, (KKKEE)₃K, (KKKEEE)₃K und (KKEEE)₃K.

Ein weiterer Gegenstand der vorliegenden Erfindung ist auch ein Konjugat enthaltend mindestens ein Peptid, wie zuvor definiert, und mindestens einen kovalent, optional über einen Spacer, gebundenen Wirkstoff, wie in Anspruch 9 definiert.

Erfindungsgemäß können pro erfindungsgemäßem Konjugat ein oder mehrere gleiche oder unterschiedliche Wirkstoffmoleküle gebunden sein.

Genauso kann das erfindungsgemäße Konjugat, insbesondere bei Makromolekülen wie größeren Wirkstoffmolekülen, beispielweise Proteinen, auch zwei oder mehrere Peptide enthalten, die an ein Wirkstoffmolekül gebunden sind, um eine nierenspezifische Anreicherung des Wirkstoffs zu ermöglichen. Typischerweise erfolgt auch hier eine kovalente Anbindung der Peptide an das Makromolekül. Als Makromoleküle gelten erfindungsgemäß nicht nur große Moleküle wie Proteine sondern auch jede Form von Partikeln (z.B. Nanopartikel), Liposomen oder andere Systeme, mit denen Wirkstoffe transportiert oder an die Wirkstoffe gebunden sein können.

Unter einem Wirkstoff wird erfindungsgemäß jede Substanz verstanden, die an das Oligomer gekoppelt werden kann, um es für die diagnostische und/oder therapeutische Behandlung einzusetzen. Erfindungsgemäß kann es sich dabei also sowohl um ein Signalmolekül, als auch um einen "klassischen" Wirkstoff handeln.

Erfindungsgemäß sind Wirkstoffe oder Wirkstoffmoleküle entsprechend dem deutschen Arzneimittelgesetz Stoffe, die dazu bestimmt sind, bei der Herstellung von Arzneimitteln als arzneilich wirksame Bestandteile verwendet zu werden oder bei ihrer Verwendung in der Arzneimittelherstellung zu arzneilich wirksamen Bestandteilen zu werden (AMG § 4 (19)). Wirkstoffe rufen in der Regel in einem Organismus eine spezifische Wirkung hervor. Ein erfindungsgemäßer Wirkstoff ist typischerweise ein pharmazeutisch wirksames Molekül oder Arzneimittel, wie etwa **Immunsuppressiva** z. B. Azathioprin, Mycophenolat-Mofetil, Ciclosporin, Tacrolimus, Sirolimus, Fingolimod oder Triptolid, **Zytostatika** z. B. Atrasentan, Nintedanib, Bleomycin, Dactinomycin, Mitomycin, Daunorubicin, Doxorubicin, Epirubicin, Idarubicin, Mitoxantron, Amsacrin, Doxofluridin, Cisplatin, Carboplatin, Oxaliplatin, Satraplatin, Camptothecin, Toptecan, Irinotecan, Etoposid, Teniposid, Cyclophosphamid, Trofosfamid, Melphalan, Chlorambucil, Estramustin, Busulfan, Chlorambucil, Chlormethin, Treosulfan, Carmustin, Lomustin, Nimustin, Procarbazin, Streptozocin, Dacarbazin, Ifosfamid, Temozolomid, Thiotepa, Vinorelbin, Vincristin, Vinblastin, Vindesin, Paclitaxel, Docetaxel, Methotrexat, Pemetrexed, Raltitrexed, Fluorouracil, Capecitabin, Cytosinarabinosid, Gemcitabin, Tioguanin, Pentostatin, Mercaptopurin, Fludarabin, Caldribin, Hydroxycarbamid, Mitotan, Azacitidin, Cytarabin, Nelarabin, Bortezomib, Anagrelid insbesondere der Protein-Kinase-Inhibitoren wie z. B. Imatinib, Erlotinib, Sunitinib, Sorafenib, Dasatinib, Lapatinib oder Nilotinib, **Immuntherapeutika** z. B. Cetuximab, Alemtuzumab und Bevacizumab, **Antiphlogistika** z. B. Naproxen, Ibuprofen, Indometacin, Prednisolon, Prednison, Hydrocortison oder Budesonid, **Antibiotika** insbesondere der Penicilline wie z. B. Benzylpenicillin, Methicillin oder Amoxicillin, der Cephalosporine wie z. B. Cefuroxim, Cefotaxim, Cefadroxil oder Cefixim, der □-Lactamase-Inhibitoren wie z. B. Clavulansäure, Sulbactam oder Tazobactam, der Carbapeneme wie z. B. Imipenem oder Meropenem, der Monobactame wie z. B. Aztreonam, der Tetracycline wie z. B. Tetracyclin, Chlortetracyclin, Oxytetracyclin, Doxycyclin, Minocyclin oder Tigecyclin, der Makrolid-Antibiotika wie z. B. Erythromycin A, der Glykopeptid-Antibiotika wie z. B. Vancomycin, der Endiine wie z.B. Calicheamicin, **Virostatika** z. B. Aciclovir, Valaciclovir, Ganciclovir, Valganciclovir, Penciclovir, Famciclovir, Brivudin, Cidofovir, Foscarnet, Idoxuridin oder Tromantadin, **Antihypertensiva** insbesondere der **ACE-Hemmer** wie z. B. Benazepril, Captopril, Cilazapril, Enalapril, Fosinopril, Lisinopril, Perindopril, Quinapril, Ramipril, Trandolapril oder Zofenopril, der **Sartane** wie z. B. Losartan, Balsartan, Irbesartan, Candesartan, Eprosartan, Olmesartan oder Telmisartan, der **Renin-Inhibitoren** wie z. B. Aliskiren und der **Betablocker** wie z. B. Proproanolol, Pindolol, Sotalol, Bopindolol, Atenolol, Bisorpolol, Celiprolol, Esmolol, Metoprolol, Nebivolol, Oxprenolol, Carvedilol oder Labetalol, **Urikosurika** z. B. Probenecid oder Benzbromaron oder **Diuretika** z. B. Acetazolamid, Furosemid, Torasemid, Bumetanid, Piretanid, Azosemid, Etacrynsäure, Etozolin, Hydrochlorothiazid, Benzthiazid, Chlorothiazid, Chlorthalidon, Indapamid, Mefrusid, Metolazon, Clopamid, Xipamid, Hydroflumethiazid, Methyclothiazid, Polythiazid, Amilorid, Triameteren, Spironolacton, Canrenon, Eplerenon oder Spironolacton, **Antifibrotika** z. B. Pirfenidon oder Seliciclib.

Weitere Antitumormittel, z. B. Mittel, die gegen proliferierende Zellen wirksam sind, sind erfindungsgemäß ebenfalls Wirkstoffe. Beispielhafte Antitumormittel schließen Cytokine wie etwa Interleukin-2 (IL-2), Tumornekrosefaktor oder dergleichen, Lectin-Entzündungsreaktionspromotoren (Selectine) wie etwa L-Selectin, E-Selectin, P-Selectin oder dergleichen, und ähnliche Moleküle ein.

Zusätzlich zu den Wirkstoffmolekülen oder statt der Wirkstoffmoleküle können an das erfindungsgemäße Konjugat auch andere Funktionalitäten, wie beispielsweise Funktionalitäten für diagnostische oder bildgebende Verfahren, gebunden sein.

Genauso können als Funktionalität über optionale Spacer fluorhaltige Seitenketten eingebaut werden. Mit Hilfe der ¹⁹F-Kernresonanztomographie kann so die Anreicherung der entsprechenden Moleküle in den Nieren dargestellt werden. Besonders vorteilhaft sind dabei hochsymmetrisch angeordnete Fluoratome, die eine einheitliche Resonanzfrequenz aufweisen. Zur Verbesserung des ¹⁹F-Signals kann ein in der Kernspintomographie übliches Kontrastmittel wie beispielsweise Gadobutrol (Magnevist®), verwendet werden.

Ebenso können Komplexbildner als "Wirkstoff" im Konjugat enthalten sein. Ein Komplexbildner ist erfindungsgemäß jede Molekülstruktur, die in der Lage ist, Metallionen zu komplexieren, d.h. mit den Metallionen einen Metall-Chelat-Komplex zu bilden. Komplexbildner werden oft auch als Chelatoren bezeichnet. Beispiele für erfindungsgemäß geeignete Komplexbildner sind EDTA, NOTA, TETA, Iminodiessigsäure, DOTA oder DTPA. Besonders bevorzugt sind erfindungsgemäß Komplexbildner, die Metallionen binden, die in SPECT, PET, CT oder MRT-Messungen detektiert werden können. Bevorzugte Komplexbildner sind DOTA oder DTPA oder deren Derivate. Komplexbildner sind erfindungsgemäß sowohl Moleküle, an die die Metallionen bereits gebunden sind, wie auch Moleküle, an die Metallionen binden können, aber im vorliegenden Stadium nicht gebunden sind.

Erfindungsgemäß geeignete Metallionen zur Bindung an Komplexbildner sind z.B. Fe²⁺, Fe³⁺, Cu²⁺ , Cr³⁺, Gd³⁺, Eu³⁺, Dy³⁺, La³⁺, Yb³⁺ und/oder Mn²⁺ oder auch die Ionen von Radionukliden, wie Gamma-Emitter, Positronen-Emitter, Auger-Elektronen-Emitter, Alpha-Strahler und Fluoreszenz-Emitter, z.B. ⁵¹Cr, ⁶⁷Ga, ⁶⁸Ga, ¹¹¹In, ^{99m}Tc, ¹⁴⁰La, ¹⁷⁵Yb, ¹⁵³Sm, ¹⁶⁶Ho, ⁸⁸Y, ⁹⁰Y, ¹⁴⁹Pm, ¹⁷⁷Lu, ⁴⁷Sc, ¹⁴²Pr, ¹⁵⁹Gd, ²¹²Bi, ⁷²As, ⁷²Se, ⁹⁷Ru, ¹⁰⁹Pd, ¹⁰⁵Rh, ^{101m}Rh, ¹¹⁹Sb, ¹²⁸Ba, ¹⁹⁷Hg, ²¹¹At, ¹⁶⁹Eu, ²⁰³Pb, ²¹²Pb, ⁶⁴Cu, ⁶⁷Cu, ¹⁸⁸Re, ¹⁸⁶Re, ¹⁹⁸Au und/oder ¹⁹⁹Ag.

Beispiele für geeignete Metallionen und ihre jeweilige Anwendung sind:
- ¹¹¹In für SPECT
- ⁶⁸Ga für PET
- ⁹⁰Y zur Therapie
- Gd, Eu, Mn für MRT
- Tantal, Wolfram oder andere Elemente mit hoher Ordnungszahl für die Computertomographie

Falls das erfindungsgemäße Konjugat Komplexbildner enthält, ist es besonders vorteilhaft Gadolinium oder Mangan oder ein anderes dem Fachmann bekanntes stark paramagnetisches Metallion mit Hilfe eines am erfindungsgemäßen Konjugat befindlichen Komplexbildners zu integrieren. Geeignete Komplexbildner sind dabei beispielsweise DOTA und DTPA.

Weiterhin können - auch optional über Spacer - Komplexbildner konjugiert werden, wie Hydroxychinolin-, Thioharnstoff-, Guanidin-, Dithiocarbamat-, Hydroxamsäure-, Amidoxim-, Aminophosphorsäure-, (cyclische) Polyamino-, Mercapto-, 1,3-Dicarbonyl- und Kronenether-Reste mit zum Teil sehr spezifischen Aktivitäten gegenüber Ionen unterschiedlicher Metalle.

Auch können Funktionalitäten für zellspezifisches Targeting wie z. B. Antikörper, Antikörperfragmente oder Aptamere an das erfindungsgemäße Konjugat gebunden sein. Auch Fluoreszenzfarbstofffe oder Interleukine wie IL-2 können gebunden sein.

Wirkstoffe, Peptide, Komplexbildner oder andere Funktionalitäten können direkt oder mittels eines Spacers kovalent an das Peptid gebunden sein.

Ein Spacer, oft auch als Linker bezeichnet, bewirkt eine kovalente Bindung zwischen zwei Teilen eines Moleküls, im vorliegenden Fall beispielsweise zwischen dem Peptid und einem Wirkstoff. Ein Spacer wird beispielsweise dann eingeführt, wenn die Verbindung zwischen zwei Molekülteilen nicht nur über eine direkte chemische Bindung erfolgen soll, sondern ein gewisser Abstand zwischen zwei Molekülteilen erzeugt werden soll. Genauso kann ein Spacer die chemischen Funktionalitäten zur Verfügung stellen, die notwendig sind, um zwei Teile eines Moleküls, die ansonsten nicht miteinander reagieren würden, zu verbinden. Bevorzugt erfolgt die Konjugation eines Spacers an das Peptid oder einen Wirkstoff über eine Amid- oder eine Esterbindung. Spacer können beispielsweise aliphatische Kohlenwasserstoffe, Polyether (wie Polyethylenglycole), Peptide oder ähnliche Elemente mit Ketten-Struktur sein. Der Spacer kann stabil sein, d.h. er ist unter physiologischen Bedingungen nicht oder nur geringfügig spaltbar, oder er kann instabil sein, d.h. er ist zumindest unter bestimmten physiologischen Bedingungen spaltbar.

Beispiele für funktionelle Gruppen über die eine direkte Anbindung erfolgen kann sind -NH₂, -SH, -OH, -Hal (z.B. -Cl, -Br, -I), -Alkin, -NCS, -NCO, SO₂Cl, -Azid, -Carbonat, -Aldehyd, -Epoxid, -COOH, -COOR, wobei R in diesem Fall bevorzugt ein Halogen oder bevorzugt ein Aktivator, d.h. eine gute Abgangsgruppe ist, z.B. *N*-Hydroxysuccinimid, Pentafluorphenyl oder para-Nitrophenyl. Einen Überblick über mögliche kovalente Kopplungsarten findet man z.B. in "Bioconjugate Techniques", Greg T. Hermanson, Academic Press, 1996 auf den Seiten 137 bis 165.

Beispielsweise können beim erfindungsgemäßen Konjugat Wirkstoffe über einen spaltbaren Linker gebunden sein. Dieser Linker wird dann unter bestimmten Bedingungen, z.B. enzymatisch oder chemisch, *in vivo* gespalten und setzt den Wirkstoff frei. Für diesen Zweck sind Linker, die Carbonsäureester- und Disulfidbindungen enthalten, geeignet, worin die ersteren Gruppen enzymatisch oder chemisch hydrolysiert werden und die letzteren durch Disulfidaustausch, z. B. in Gegenwart von Glutathion, abgetrennt werden.

Ein Beispiel für einen spaltbaren Spacer ist auch ein Peptid, welches gezielt mit Hilfe von speziellen, körpereigenen oder aber auch dem Körper zugesetzten Enzymen gespalten werden kann. So wird z.B. die Peptidsequenz DEVD (Asp-Glu-Val-Asp) nach Apoptoseinduktion durch Caspase-3 gespalten. Damit kann beispielsweise ein Wirkstoff der über einen derartigen Spacer gebunden ist, nach einer bestimmten Verweilzeit in der Niere aus selbiger entfernt werden, oder aber auch eine entsprechende Funktionalität (Vorhandensein oder Abwesenheit eines bestimmten Enzyms) der Niere überprüft werden. Weitere Beispiele sind die Peptidsequenzen CPEN↓FFWGGGG (Salinas et al. 2008, Biomaterials 29, 2370-2377) oder PENFF, die durch die Matrix-Metalloprotease-13 gespalten werden kann.

Eine einfache Ausführungsform eines spaltbaren Spacers ist die Bildung eines Carbonsäureesters, der durch Esterasen leicht gespalten werden kann.
Daher ist in einer bevorzugten Ausführungsform der vorliegenden Erfindung der Wirkstoff über eine Esterverknüpfung gebunden. Dies ermöglicht eine exakte Abspaltung des Wirkstoffmoleküls in der Niere. Gleichzeitig ist die Verknüpfung jedoch zuvor für den Transport in die Niere ausreichend stabil, um eine vorzeitige Abspaltung zu vermeiden.
Weiterhin ermöglicht eine leicht spaltbare Esterverknüpfung des Wirkstoffes mit dem Wirkstofftransporter, eine relativ schnelle Freisetzung des Wirkstoffs am Zielort. Die Spaltung der Esterverknüpfung erfolgt zeitlich schneller als der Abbau des Wirkstofftransporters durch Proteasen.

Alternativ kann der Spacer eine säurelabile Struktur, z.B. ein Hydrazon, ein Imin, ein Carboxylhydrazon, ein Acetal oder Ketal enthalten (siehe beispielsweise Haag-R, Kratz-F, Angewandte Chemie Seite 1218 (2006)).

Zur Konjugation von Wirkstoffen mit aliphatischen oder aromatischen Hydroxylgruppen sind Carbonate vorteilhaft. Sie lassen sich aus den entsprechenden Alkoholen bzw. Phenolen durch Umsetzung mit Chlorformiaten einfach und in hoher Ausbeute synthetisieren. Besonders vorteilhaft ist dabei die Umsetzung mit Chlorformiaten, die eine Dreifachbindung enthalten, wie beispielsweise Propargylchlorformiat (CAS-Nummer 35718-08-2). Durch die so eingeführte Dreifachbindung lassen sich die enzymatisch leicht spaltbaren Carbonate mit Aziden, die in das Peptidoligomer eingebaut sind, mittels 1,3-dipolarer Cycloaddition, der sogenannten Huisgen-Reaktion, einfach und unter sehr schonenden Bedingungen verknüpfen.

Das gleiche gilt für die Konjugation von aliphatischen oder aromatischen Aminogruppen mit Hilfe von Chlorformiaten. Hierbei bilden sich statt der Carbonate die entsprechenden Carbamate, die ebenfalls durch Esterasen leicht spaltbar sind. Auch hier ist die Verknüpfung mit Chlorformiaten, die eine Dreifachbindung enthalten, besonders vorteilhaft.

Erfindungsgemäß kann der mindestens eine Wirkstoff an den N- und/oder C-Terminus des Peptids gebunden sein.
In einer alternativen Ausführungsform kann der Wirkstoff an eine Aminosäure in der Kette gebunden sein.
In einer weiteren alternativen Ausführungsform kann der Wirkstoff in der Kette zwischen den Aminosäuren gebunden sein.

Das erfindungsgemäße Peptid und dessen Wirkstoff-Konjugat werden hochselektiv von den Nieren aufgenommen und relativ rasch abgebaut. Durch die geeignete Auswahl der Kettenlänge und des molekularen Aufbaus des Peptids, sowie durch die geeignete Auswahl des Verknüpfungsortes des Wirkstoffs am Peptid, lässt sich dabei die gewünschte Pharmakokinetik, d.h. die gewünschte Wirkstofffreisetzung am Zielort, d.h. in der Niere, einstellen.

Typischerweise führen längere Peptide zu einer verzögerten Freisetzung verglichen mit kürzeren Peptiden. Längere Peptide haben beispielsweise Kettenlängen von 20 bis 40 Aminosäuren, bevorzugt 30 Aminosäuren, während unter kürzeren Peptiden typischerweise Kettenlängen von 3 bis 10 Aminosäure, bevorzugt 5 Aminosäuren, verstanden werden.

Die Freisetzung C-terminal-verknüpfter Wirkstoffe erfolgt wesentlich schneller als die von N-terminal-verknüpften Wirkstoffen. Ohne an diese Theorie gebunden zu sein, wird vermutet, dass der geschwindigkeitsbestimmende Schritt beim Peptidabbau vor allem durch Carboxypeptidasen beeinflusst wird, die vom C-Terminus aus beginnend das Peptid abbauen.

Erfindungsgemäß werden auch verzweigt in der Kette eingebaute Wirkstoffe deutlich langsamer freigesetzt, als linear verknüpfte. Der enzymatische Abbau verzweigter Peptidstrukturen ist grundsätzlich deutlich schwieriger als der Abbau linearer Peptide.

Weiterhin lässt sich erfindungsgemäß die Freisetzungsgeschwindigkeit des Wirkstoffs auch durch die Art dessen Verknüpfung an das Oligomer steuern. Eine leicht spaltbare Esterverknüpfung ermöglicht eine relativ schnelle Freisetzung des Wirkstoffs am Zielort (siehe oben).

Gegenstand der vorliegenden Erfindung ist auch ein Verfahren zur Herstellung eines Konjugats, wie zuvor beschrieben, dadurch gekennzeichnet, dass ein optional aktivierter Wirkstoff an das Peptid konjugiert wird.

Die Herstellung der erfindungsgemäßen Konjugate weist typischerweise zumindest die folgenden Verfahrensschritte auf:
a) Bereitstellen eines erfindungsgemäßen Peptids, das zumindest eine reaktive Gruppe aufweist,
b) Konjugation mindestens eines optional aktivierten Wirkstoffs an das Peptid aus Schritt a).

In einer Ausführungsform des erfindungsgemäßen Verfahrens, falls das Konjugat als Wirkstoff einen Komplexbildner enthält, wird in einem weiteren Schritt c) die in Schritt b) erhaltene Verbindung mit Metallsalzen kontaktiert, so dass Metallionen von den Komplexbildnern komplexiert werden.

Die Peptide der erfindungsgemäßen Konjugate können insbesondere auf verschiedene, dem Fachmann im Bereich der Peptidsynthese bekannte, Verfahren hergestellt werden.

Typischerweise erfolgt die Herstellung über eine Festphasensynthese. Eine Festphase ist erfindungsgemäß ein organisches, anorganisches oder organisch/anorganisches Verbundmaterial, das als Harz oder Träger in der Festphasensynthese eingesetzt werden kann. Des Weiteren gelten erfindungsgemäß als Festphase auch Oberflächen von Formkörpern wie z.B. Mikrotiterplatten oder partikuläre Materialien, wie z.B. organische oder anorganische Nanopartikel, Metallpartikel oder ähnliches.

Die Festphasensynthese wird entsprechend einer herkömmlichen Peptid-Synthese durchgeführt (z.B. Fmoc/*t*Bu-Peptid-Synthese oder Boc/Benzyl-Peptid-Synthese). Dem Fachmann sind derartige Festphasensynthesen bekannt. Geeignete Lehrbücher für die Peptidsynthese sind "Solid-Phase Peptide Synthesis": 289 (Methods in Enzymology) von Sidney P. Colowick (Autor), Gregg B. Fields (Herausgeber), Melvin I. Simon (Herausgeber) Academic Press Inc (November 1997) oder "Fmoc Solid Phase Peptide Synthesis: A Practical Approach" von W. Chan (Autor), W. C. Chan (Herausgeber), Peter D. White (Herausgeber) Oxford Univ Pr (2. März 2000). Die jeweils eingesetzten Monomere werden dabei so gewählt, dass ein Peptid entsprechend der vorliegenden Erfindung entsteht. Je nach Art der Aminosäureeinheit kann zur Synthese direkt eine derivatisierte Aminosäureeinheit verwendet werden oder eine zunächst an der für die Derivatisierung vorgesehenen Stelle geschützte Aminosäureeinheit. Nach erfolgter Synthese des Peptids kann dann entweder noch an der Festphase oder nach Abspaltung von der Festphase in Lösung die abschließende Derivatisierung mit dem Wirkstoff vorgenommen werden.

Die Anbindung des Wirkstoffs erfolgt in diesem Fall bevorzugt an das fertige Peptid, d.h. entweder nach erfolgter Festphasensynthese des Peptids noch an der Festphase oder nach dessen Abspaltung in Lösung.

Soll der Wirkstoff beispielsweise an das N-terminale Ende des Peptids gebunden werden, werden die Peptide typischerweise mit einer Aminoterminalen Schutzgruppe erzeugt, wie etwa Fmoc. Sofern der Wirkstoff die Bedingungen überstehen kann, die verwendet werden, um einerseits das Peptid vom Syntheseharz abzuspalten und andererseits die Seitenketten zu entschützen, kann die Fmoc-Gruppe vom N-Terminus des vollständigen Harz-gebundenen Peptids abgespalten werden, so dass der Wirkstoff an das freie N-terminale Amin gebunden werden kann. In solchen Fällen wird der Wirkstoff typischerweise durch Verfahren aktiviert, die in der Technik allgemein dafür bekannt sind, dass sie eine aktive Ester- oder aktive Carbonatgruppe erzeugen, die wirkungsvoll ist, um eine Amid- bzw. Carbamatbindung mit der Oligomer-Aminogruppe zu bilden. Natürlich kann auch eine andere Verknüpfungschemie verwendet werden.

Um Nebenreaktionen zu minimieren, können Guanidino- und Amidinogruppen unter Verwendung herkömmlicher Schutzgruppen, wie etwa Carbobenzyloxygruppen (CBZ), di-*t*-BOC, PMC, Pbf, N-NO₂ und ähnlichen blockiert werden.

Kopplungsreaktionen werden durchgeführt durch bekannte Kopplungsverfahren in Lösungsmitteln, wie etwa N,N-Dimethylformamid (DMF), *N*-Methylpyrrolidon, Dichlormethan und/oder Wasser. Beispielhafte Kopplungsreagenzien umfassen O-Benzotriazolyloxytetramethyluroniumhexafluorphosphat (HATU), Dicyclohexylcarbodiimid, Brom-tris(pyrrolidino)phosphoniumbromid (PyBroP), usw. Andere Reagenzien können enthalten sein, wie etwa *N,N-*Dimethylaminopyridin (DMAP), 4-Pyrrolidinopyridin, *N*-Hydroxysuccinimid oder N-Hydroxybenzotriazol.

Enthält das Molekül Komplexbildner können die Metallionen nach bekannten Methoden komplexiert werden.

Die vorliegende Erfindung basiert auf dem überraschenden Effekt, dass die erfindungsgemäßen Peptide und Konjugate z.B. nach Injektion in die Blutbahn oder nach subkutaner Injektion nahezu ausschließlich in der Niere angereichert werden. Dementsprechend sind die erfindungsgemäßen Peptide und/oder Konjugate für den Einsatz in therapeutischen Verfahren zur Behandlung der Niere, in bildgebenden Verfahren zur Darstellung der Niere sowie zum Nieren-Targeting geeignet.

Gegenstand der vorliegenden Erfindung ist daher auch ein erfindungsgemäßes Peptid oder Konjugat, wie zuvor beschrieben, zur Verwendung als Arzneimittel, wie insbesondere einer therapeutischen Zusammensetzung oder einer bildverstärkenden Zusammensetzung.

Gegenstand der vorliegenden Erfindung ist das Peptid oder Konjugat, wie zuvor beschrieben, zur Verwendung zum Targeting der Niere. Dabei dient das Targeting der Niere bevorzugt dazu, Arzneimittel für pharmazeutische oder diagnostische Anwendungen in der Niere anzureichern, d.h. im Verhältnis zum übrigen Körper eine vermehrte Aufnahme in der Niere zu erzeugen.

Alternativ kann auch das Peptid allein ohne einen gebundenen Wirkstoff in der Niere angereichert werden. Aufgrund seiner hohen Selektivität für die Niere kann daher durch Verabreichung des Peptids beispielsweise die Anreicherung von nierenschädigenden Stoffen, die im Rahmen einer Therapie verabreicht werden, vermieden oder zumindest reduziert werden.

Gegenstand der vorliegenden Erfindung ist daher auch das Peptid wie zuvor beschrieben zur Verwendung zum Schutz der Niere.

In der Radiopeptidtherapie von neuroendokrinen Tumoren wird beispielsweise die Substanz DOTATOC verwendet. Dieses mit DOTA konjugierte Octapeptid hat die unerwünschte Nebenwirkung, dass es zu ca. 20 % von den Nieren (sprich proximalen Tubuluszellen, PTC) aufgenommen wird. Dabei sind die Schäden in den Nieren Dosis-Therapiezyklen-limitierend. Die Aufnahme von DOTATOC kann gesenkt werden, wenn zeitgleich das erfindungsgemäße Peptid verabreicht wird. Ohne an die Theorie gebunden zu sein wird vermutet, dass dabei der für die Aufnahme des DOTATOCs verantwortliche Rezeptor (Megalin/Cubilin) auf der apikalen Seite der PTCs blockiert wird und deshalb mehr DOTATOC in den Urin geht.

Die Verwendung der erfindungsgemäßen Konjugate zum Targeting der Niere ist im Vergleich zu anderen bekannten niedermolekularen Strukturen vorteilhaft, da diese auch in der Konjugation mit dem Wirkstoff eine sehr gute Nierenanreicherung zeigen. Der Vergleich mit in der Literatur beschriebenen Peptiden, die selektiv von den Nieren aufgenommen werden (APASLYN und HITSLLS, Aminosäuren sind im Einbuchstaben-Code angegeben (Denby et al.: Molecular Therapy 15, 9, 2007, 1647-1654)), ergibt, dass zwar die meisten Peptide eine mehr oder minder stark ausgeprägte Nierenselektivität nach der intravenösen Applikation aufweisen, jedoch nicht in der Konjugation mit einem Wirkstoff.
Der pharmakologische Nutzen der Peptidstrukturen als Transportsystem für die Behandlung von Nierenerkrankungen ist jedoch nur dann gegeben, wenn diese Peptide zusammen mit konjugierten Wirkstoffen nahezu ausschließlich von den Nieren, namentlich den proximalen Tubuluszellen, aufgenommen werden. Nur so ergibt sich ein signifikanter Vorteil gegenüber der systemischen Gabe des Wirkstoffes.

Weiterhin ermöglichen die erfindungsgemäßen Konjugate, dass neben der in der Literatur beschriebenen intravenösen Gabe von Peptiden/Proteinen zum Wirkstofftransport in die Nieren auch die subkutane und intraperitoneale Gabe der erfindungsgemäßen Peptid-Wirkstoff-Konjugate erfolgreich die Nieren adressieren kann.

Die intraperitonale, und speziell die subkutane Administrationsroute, ist für die Applizierung eines potentiellen Wirkstoffes, gegenüber der intravenösen Route, für Arzt und Patient vorteilhaft.

Gegenstand der vorliegenden Erfindung ist auch ein Arzneimittel bzw. eine pharmazeutische Zusammensetzung, insbesondere eine therapeutische oder eine bildverstärkende Zusammensetzung, enthaltend mindestens ein erfindungsgemäßes Peptid oder Konjugat, wie zuvor beschrieben.

Erfindungsgemäß kann das Peptid oder Konjugat auch in Form seiner pharmazeutisch verwendbaren Salze und Stereoisomere, einschließlich deren Mischungen in allen Verhältnissen, vorliegen.

Erfindungsgemäß ist auch die Verwendung der erfindungsgemäßen Peptide und/oder Konjugate zur Herstellung einer pharmazeutischen Zusammensetzung bzw. eines Arzneimittels, insbesondere einer therapeutischen Zusammensetzung, und/oder einer bildverstärkenden Zusammensetzung (z.B. ein Kontrastmittel) und/oder eines radiomarkierten Tracers für die nuklearmedizinische Bildgebung.

Erfindungsgemäß kann es sich bei der vorliegenden Erfindung auch um ein Kit zur Herstellung eines Arzneimittels bzw. einer pharmazeutischen Zusammensetzung handeln, insbesondere einer therapeutischen oder bildverstärkenden Zusammensetzung, enthaltend mindestens ein erfindungsgemäßes Peptid und/oder Konjugat. Dieses Peptid und/oder Konjugat kann dann je nach Anwendungszweck beispielsweise mit einem geeigneten Wirkstoff umgesetzt werden zur Herstellung einer therapeutischen oder bildverstärkenden Zusammensetzung.
Als bildverstärkende Zusammensetzung oder Kontrastmedium bzw. bildverstärkend wirkende Stoffe, werden erfindungsgemäß Stoffe bzw. Zusammensetzungen verstanden, die in bestimmten diagnostischen Verfahren die Darstellung des Zielorgans verbessern, in der Regel indem der Kontrast zur Umgebung bzw. das Signal des Zielorgans im Verhältnis zur Umgebung erhöht wird.

Die vorliegende Erfindung betrifft zudem die erfindungsgemäßen Peptide und/oder Konjugate, und/oder deren pharmazeutisch verwendbaren Salze und Stereoisomere, einschließlich deren Mischungen in allen Verhältnissen, sowie gegebenenfalls Träger- und/oder Hilfsstoffe
- als Arzneimittel
- zur Verwendung als Arzneimittel
- als Wirkstoff oder wirksame Komponente in einem Arzneimittel
- als Diagnostikum
- zur Verwendung als Diagnostikum
- zur Verwendung beim Targeting der Niere
- sowie insbesondere als Arzneimittel zur Behandlung von Erkrankungen der Niere.

Eine therapeutische Zusammensetzung, eine pharmazeutische Zusammensetzung oder ein Arzneimittel besteht in der Regel zumindest aus dem Wirkstoff - in diesem Fall dem erfindungsgemäßen Peptid oder Konjugat mit dem angebundenem Wirkstoff - und einem oder mehreren geeigneten Lösungsmitteln und/oder Trägerstoffen, die die Applikation der therapeutischen Zusammensetzung erlauben.

Eine diagnostische Zusammensetzung bzw. ein Diagnostikum dient in diagnostischen Verfahren als bildverstärkende oder bildgebende Zusammensetzung. Ein Diagnostikum besteht in der Regel zumindest aus der Signalquelle, d.h. aus der bildgebenden und/oder bildverstärkenden Komponente - in diesem Fall dem erfindungsgemäßen Konjugat, wobei in diesem Fall bevorzugt mindestens ein Wirkstoff ein Komplexbildner ist- und einem oder mehreren geeigneten Lösungsmitteln und/oder Trägerstoffen, die die Applikation der diagnostischen Zusammensetzung erlauben.

Für diagnostische Anwendungen dient das erfindungsgemäße Konjugat bevorzugt als Signalquelle in einem bildverstärkenden Kontrastmedium, so dass dieses mittels nuklearmedizinischer und/oder radiologischer Verfahren wie SPECT, PET, Ultraschall und oder auch durch Magnetresonanztomographie, computertomographische und optische Bildgebungsverfahren (Near-Infrared-Imaging) detektiert werden kann. Dem Fachmann sind Detektionsverfahren und Anwendungen für bildverstärkende Kontrastmedien bekannt. Beispiele für geeignete Anwendungen sind die Diagnostik von Krebserkrankungen, neurologische Fragestellungen, Überprüfung des Ansprechens auf eine Therapie, Überprüfung des Grads der Schädigung einer Niere bei z.B. Autoimmunerkrankungen und die Kontrolle von Gentherapien, jedoch auch die Erkennung von zellulären Veränderungen.

Pharmazeutische Zusammensetzungen bzw. Arzneimittel oder Medikamente lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepasste pharmazeutische Formulierungen können als separate Einheiten, wie z.B. Kapseln oder Tabletten; Pulver oder Granulate; Lösungen oder Suspensionen in wässrigen oder nichtwässrigen Flüssigkeiten; essbare Schäume oder Schaumspeisen; oder Öl-in-Wasser-Flüssigemulsionen oder Wasser-in-ÖI-Flüssigemulsionen dargereicht werden.

So lässt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glycerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem essbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Calciumstearat oder Polyethylenglykol in Festform können dem Pulvergemisch vor dem Füllvorgang zugesetzt werden. Ein Sprengmittel oder Lösungsvermittler, wie z.B. Agar-Agar, Calciumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder β-Lactose, Süßstoffe aus Mais, natürliche und synthetische Gummi, wie z.B. Akazia, Traganth oder Natriumalginat, Carboxymethylcellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylcellulose, Agar, Bentonit, Xanthangummi u.ä. Die Tabletten werden formuliert, indem beispielsweise ein Pulvergemisch hergestellt, granuliert oder trockenverpresst wird, ein Schmiermittel und ein Sprengmittel zugegeben werden und das Ganze zu Tabletten verpresst wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylcellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dicalciumphosphat, vermischt wird. Das Pulvergemisch lässt sich granulieren, indem es mit einem Bindemittel, wie z.B. Sirup, Stärkepaste, Acadia-Schleim oder Lösungen aus Cellulose- oder Polymermaterialen benetzt und durch ein Sieb gepresst wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengussformen zu verhindern. Das gefettete Gemisch wird dann zu Tabletten verpresst. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpresst werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so dass eine gegebene Quantität eine vorgegebene Menge der Verbindung enthält. Sirupe lassen sich herstellen, indem die Verbindung in einer wässrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung lässt sich auch so herstellen, dass die Freisetzung verlängert oder retardiert wird, wie beispielsweise durch Beschichtung oder Einbettung von partikulärem Material in Polymere, Wachs u.ä.

Die erfindungsgemäßen Peptide oder Konjugate lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die erfindungsgemäßen Peptide oder Konjugate können auch unter Verwendung monoklonaler Antikörper als individuelle Träger, an die die Peptide oder Konjugate gekoppelt werden, zugeführt werden. Die Peptide oder Konjugate können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Poly-ε-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepasste pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepasste pharmazeutische Verbindungen können als Salben, Cremes, Suspensionen, Lotionen, Pulver, Lösungen, Pasten, Gele, Sprays, Aerosole oder Öle formuliert sein.

An die rektale Verabreichung angepasste pharmazeutische Formulierungen können in Form von Zäpfchen oder Einläufen dargereicht werden.

An die nasale Verabreichung angepasste pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepasste pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

Zu den an die parenterale Verabreichung angepassten pharmazeutischen Formulierungen gehören wässrige und nichtwässrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wässrige und nichtwässrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so dass nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Bevorzugt werden die erfindungsgemäßen Peptide oder Konjugate parenteral verabreicht.

Es versteht sich, dass die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge des erfindungsgemäßen Peptids oder Konjugates hängt von einer Reihe von Faktoren ab, einschließlich Art des gekoppelten Wirkstoffs, dem Alter und Gewicht des Patienten, dem exakten Krankheitszustand, der der Behandlung bedarf, sowie seines Schweregrads, der Beschaffenheit der Formulierung sowie dem Verabreichungsweg.

Gegenstand der vorliegenden Beschreibung ist auch ein Kit zur Herstellung einer pharmazeutischen Zusammensetzung, insbesondere einer bildverstärkenden oder therapeutischen Zusammensetzung, zumindest enthaltend ein erfindungsgemäßes Peptid oder Konjugat. Weist das Konjugat einen Komplexbildner auf, hat dieser bevorzugt noch keine Metallionen mit bildverstärkender oder therapeutischer Wirkung komplexiert. Das erfindungsgemäße Peptid oder Konjugat kann in dem Kit in einem Lösungsmittel (z.B. einem wässrigen Puffer) gelöst vorliegen oder bevorzugt als Lyophilisat.

Da die Metallionen, die vom Komplexbildner des erfindungsgemäßen Konjugats komplexiert werden, für viele Anwendungen radioaktiv sind, können pharmazeutische Zusammensetzungen, die das Konjugat enthalten, nicht beliebig lange im voraus hergestellt werden. Weiterhin sind aufgrund der Radioaktivität bei der Herstellung bestimmte Vorschriften zur Arbeitssicherheit zu beachten. Aus diesem Grund ist es erfindungsgemäß bevorzugt, einen Kit zur Verfügung zu stellen, der das erfindungsgemäße Konjugat enthält, wobei der Komplexbildner noch nicht die für die endgültige Anwendung notwendigen Metallionen komplexiert hat.

Es wurde gefunden, dass die erfindungsgemäßen Peptide oder Konjugate bereits kurze Zeit nach der Applikation spezifisch, d.h. ausschließlich bzw. fast ausschließlich in der Niere angereichert werden. Im Falle der bevorzugten intravenösen Gabe der erfindungsgemäßen Konjugate ist schon nach 5 Minuten eine Anreicherung in der Niere zu beobachten. Nach einer Stunde befinden sich mehr als 30%, bevorzugt mehr als 50%, besonders bevorzugt mehr als 70%, ganz besonders bevorzugt mehr als 80% der injizierten Dosis in der Niere (%-Angaben basieren auf Messung der Radioaktivität).

Bei Organverteilungsstudien mit radiomarkierten erfindungsgemäßen Konjugaten (beispielsweise PET-Messungen oder anderer nicht-invasiver Bildgebung) zeigen die erfindungsgemäßen Konjugate eine Stunde nach intravenöser Applikation typischerweise mindestens eine 2-fache, bevorzugt mindestens eine 5-fache, besonders bevorzugt mindestens eine 10-fache Anreicherung in der Niere im Verhältnis zum übrigen Körper (Blut, Herz, Lunge, Milz, Leber, Muskel, Gehirn). Das bedeutet, dass das Signal, das direkt mit der Menge der radiomarkierten Verbindung korreliert, in der Niere mindestens 2 mal stärker ist als die Summe der von Blut, Herz, Lunge, Milz, Leber, Muskel und Gehirn zusammen erhaltenen Signale.

Daher können die erfindungsgemäßen Peptide oder Konjugate hervorragend für diagnostische Anwendungen, wie Nierenszintigraphie, Nieren-PET und Nieren-MRT, Funktionsprüfungen der Niere allgemein, zur Therapie und Diagnostik von Nierenkrebs und gegebenenfalls Metastasen von Nierenkrebs, CT der Niere und/oder Ultraschall der Niere, sowie zum gezielten Targeting der Niere eingesetzt werden.

Die therapeutische Anwendung liegt insbesondere im Drug Targeting für das Organ Niere. Insbesondere können die erfindungsgemäßen Peptide oder Konjugate als Arzneimittel dienen zur Behandlung von Erkrankungen der Niere oder von Erkrankungen, bei deren Behandlung Arzneimittel eingesetzt werden, deren Wirkort die Niere ist. Bevorzugt werden dabei an die erfindungsgemäßen Peptide ein oder mehrere Wirkstoffe wie Antibiotika, Entzündungshemmer, ACE-Hemmer, Diuretika, Immunsuppressiva oder Chemotherapeutika, beispielsweise über spaltbare Spacer-Sequenzen, angebunden.
Auch der Einsatz der erfindungsgemäßen Konjugate zur Blockade der Rückresorption nierentoxischer Substanzen ist möglich.
Weiterhin können die erfindungsgemäßen Peptide auch eingesetzt werden, um die Aufnahme von nierenschädigenden Stoffen in die Nieren zu verhindern oder zu verringern.

Targeting der Niere bedeutet erfindungsgemäß das Erreichen einer vermehrten Aufnahme des applizierten Stoffes in der Niere im Verhältnis zum übrigen Körper. Beim Targeting der Niere mit dem erfindungsgemäßen Peptid oder Konjugat wird durch Gabe eines erfindungsgemäßen Peptids oder Konjugats bevorzugt mindestens eine 2-fache, bevorzugt mindestens eine 5-fache, besonders bevorzugt mindestens eine 10-fache Anreicherung in der Niere im Verhältnis zum übrigen Körper (Blut, Herz, Lunge, Milz, Leber, Muskel, Gehirn) erreicht. Diese Werte werden mittels Organverteilungsstudien mit radiomarkierten erfindungsgemäßen Konjugaten (beispielsweise PET-Messungen oder anderer nicht-invasiver Bildgebung) ermittelt. Die Anreicherung in der Niere erfolgt je nach Art der Applikation typischerweise nach 30 Minuten bis 8 Stunden.

### Abbildungen:

Abb. 1 zeigt den Einfluss der Kettenlänge auf die Wirkstofffreisetzung für die Strukturen MAG3-KKEEEKKEEEKKEEEK und MAG3-KKEEEKKEEEKKEEEKKEEEKKEEEKKEEE (N-terminale Verknüpfung des Wirkstoffs - Abb. 1, oben) und KKEEEKKEEEKKEEE-y und KKEEEKKEEEKKEEEKKEEEKKEEEKKEEE-y (C-terminale Verknüpfung des Wirkstoffs - Abb. 1, unten).
Abb. 2 zeigt den Einfluss der Kettenlänge auf die Wirkstofffreisetzung für die Struktur y-KKEEEKKEEEKKEEEK (N-terminale Verknüpfung des Wirkstoffs -Abb. 2, unten) und die Strukturen KKEEEKKEEEKKEEE-y und KKEEEKKEEEKKEEEKKEEEKKEEEKKEEE-y (C-terminale Verknüpfung des Wirkstoffs -Abb. 2, oben).
Abb. 3 vergleicht die Organverteilung der beiden Strukturen KKEEEKK(y)EEEKKEEE (131-lod-Tyrosin verzweigt in Kette) und KKEEEKyEEEKKEEEK (131-lod-Tyrosin linear in Kette) eine Stunde nach Verabreichung.
Abb. 4 vergleicht den Einfluss der Verknüpfungsart anhand der Strukturen y-KKEEEKKEEEKKEEEK (Verknüpfung des Wirkstoffs über Amidbindung, Abb. 4, oben) und yoKKEEEKKEEEKKEEEK (Verknüpfung des Wirkstoffs über Esterbindung, Abb. 4, unten).
Abb. 5 vergleicht die szintigraphische Verteilung der Peptide (APASLYN)₂, y(MARIA)₃, y(MARIA)₃ als Liponsäure (LA)-Konjugat und y(KKEEE)₃ als Liponsäurekonjugat im Tiermodell Maus nach verschiedenen Zeiten. Abb. 6 zeigt die szintigraphische Verteilung des Peptids y(KKQQQ)₃K-NH₂ nach Verabreichung.
Abb.7 zeigt die szintigraphische Verteilung des Peptids y(KKQQQ)₃K-NH₂ als Liponsäure-Konjugat nach Verabreichung.
Abb. 8 zeigt die szintigraphische Verteilung des Peptids (yD₈) nach intravenöser Verabreichung an NMRI-Mäuse.
Abb. 9 vergleicht die Organverteilung des 125-lod markierten Konjugates y(KKEEE)₃K in Abhängigkeit der Administrationsroute.
Abb. 10 vergleicht die szintigraphische Verteilung der mit lod-125 radioaktiv markierten Peptide y(KKEE)₅K (Abb. 10a), y(KKKEE)₃K (Abb. 10b) und y(RREEE)₃R (Abb. 10c) jeweils 1 Stunde nach intravenöser Applikation in NMRI-Mäusen.
Abb. 11 zeigt die szintigraphische Verteilung des N-terminal gebundenen Diacetylkaffeesäure- (KKEEE)₃K Wirkstoffkonjugats nach intravenöser Applikation in einer NMRI-Maus.
Abb. 12 zeigt die szintigraphische Verteilung des zweifach konjugierten Moleküls yKKK(DCA)EEEKKEEEKKK(DCA)EEEK (CDA=Diacetylkaffeesäure) nach intravenöser Applikation in einer NMRI-Maus.
Abb. 13 zeigt die szintigraphische Verteilung von ¹²⁵I-y(KKKε(Liponsäure)EEE)₃K nach intravenöser Applikation in einer NMRI-Maus.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

### Beispiele

### 1. Materialsynthesen

### 1.1 Festphasenpeptidsynthese

Die Peptide werden am vollautomatischen Peptidsynthesizer ABI 433A der Firma Applied Biosystems GmbH (Carlsbad, CA, USA) nach der Fmoc/*t*Bu-Strategie unter Verwendung von Tentagel S RAM Harz (Beladungsgrad: 0,24 mmol/g; Rapp Polymere, Tübingen, Deutschland) als polymerer Träger hergestellt. Fmoc-Aminosäuren (Fmoc-AS-OH; Novabiochem, Merck KGaA, Darmstadt, Deutschland) mit säurelabilen Seitenkettenschutzgruppen (z. B. Arg(Pbf), Asn(Trt), Asp(O*t*Bu), Cys(Trt), Gln(Trt), Glu(O*t*Bu), His(Trt), Lys(Boc), Ser(*t*Bu), Thr(*t*Bu), Tyr(*t*Bu))werden als Ausgangsmaterialien verwendet. Der Synthesecyclus besteht aus a) Abspaltung der Fmoc-Schutzgruppe mit 20%igem Piperidin in *N*-Methyl-2-pyrrolidon (NMP), b) Waschschritte mit NMP, c) Kupplung: Fmoc-AS-OH/2-(1H-Benzotriazol-1-yl)-1,1,3,3-tetramethyluroniumhexafluorphosphat (HBTU) /Diisopropylethylamin (DIPEA)/Peptidharz 10/10/20/1, 8 Min, d) Waschschritte mit NMP. Die Effektivität der Fmoc-Abspaltungen werden mittels automatisierten Leitfähigkeitsmessungen kontrolliert. Die Peptide werden mit Trifluoressigsäure (TFA)/H₂O/Triisopropylsilan (TIPS) (95/2,5/2,5) vom Harz abgespalten (2 h bei Raumtemperatur), in kaltem Methyl-*tert*.-Butylether (MTBE) ausgefällt, mittels Zentrifugation (4000 rpm, 5 min) aufgetrennt, im Vakuum getrocknet und aus Acetonitril/H₂O (1 : 1) lyophilisiert.

MAG3 ist ein Peptidfragment aus 3 Glycineinheiten und einem Thioglykolsäurederivat, welches am Peptidsynthesizer, wie oben beschrieben, hergestellt wird (d.h. die gewünschte Peptidsequenz wird um die Einheit MAG3 verlängert).

### 1.2 Reinigung und Charakterisierung von Peptiden

Die Reinigung des vom Harz abgespaltenen Peptids mittels semipräparativer HPLC wird mit einer LaPrep-Anlage (VWR GmbH, Darmstadt, Deutschland) durchgeführt. Als Säule wird eine Waters XBridge BEH130 PREP C18 (5 µm, 19 x 150 mm) Säule verwendet (Flussraten: 8 - 20 ml/min; Lösungsmittel: 0,1 % TFA in Wasser zu 0,1 % TFA in Acetonitril). Zur Trennung wird ein Gradient von Wasser auf Acetonitril verwendet, der den physikochemischen Eigenschaften der entsprechenden Peptide angepasst wird. Das aufgereinigte Peptid wird nach Lyophilisierung erhalten.

Zur Charakterisierung werden die hergestellten Peptide mittels analytischer HPLC (Agilent 1100) und HPLC-MS (Exactive, Thermo Fisher Scientific) analysiert. Die HPLC-Analyse unter Standardbedingungen erfolgt anhand eines linearen Gradienten von 0,1 % TFA in Wasser auf 0,1 % TFA in Acetonitril in 5 min (Bedingungen: ChromolithR Performance RP-18e-Säule, 100 x 3 mm; Flussrate: 2 ml/min, Wellenlänge = 214 nm). Für die Massenspektrometrie dient ein Agilent 1200 als HPLC-System (Bedingungen: Hypersil Gold C18 Säule, 0.21 x 200 mm, Gradient: von 0,05 % TFA in Wasser auf 0,05 % TFA in Acetonitirl in 30 min, Flussrate: 200 µl/min, Säulenofen: 60 °C, Wellenlänge = 214 nm).

### 1.3 Radioaktive lodierung von Peptiden

Zur Markierung wird eine 1 mM Stammlösung des zu markierenden Peptids in Wasser (evtl. muss Dimethylsulfoxid (DMSO) zur besseren Löslichkeit zugesetzt werden) verwendet. Tyrosin-haltige Peptide werden mit lod-123, lod-125 oder lod-131 (Perkin-Elmer, Waltham, MA, USA) mittels der Chloramin-T-Methode markiert. Dazu werden 10 µl der Stammlösung mit 20 µl Phosphatpuffer (0,25 M, pH 7,4) versetzt und die gewünschte Menge an radioaktivem lod zugegeben. Zur Markierung werden 5 µl Chloramin-T (2 mg/ml H₂O) hinzugefügt. Die Reaktion erfolgt für 30 Sekunden und wird anschließend mit 10 µl einer gesättigten Methionin-Lösung abgebrochen. Um freies lod und Nebenprodukte abzutrennen, wird das Reaktionsgemisch mittels semi-präparativer HPLC (Chromolith RP-18e, 100 x 4.6 mm) gereinigt. Zur Trennung wird ein linearer Gradient von 0,1 % TFA in Wasser auf 0,1 % TFA in Acetonitril in 10 Minuten verwendet (Flussrate: 2 ml/min, UV-Absorption bei 214 nm, γ-Detektion). Anschließend wird das Lösungsmittel am Rotationsverdampfer entfernt und das markierte Peptid im gewünschten Puffer aufgenommen.

### 1.4 Radioaktive Markierung von MAG3 mit ^{99m}Technetium

Für die Markierung werden 10 µl einer 1 mM Peptidlösung zu 10 µl Phosphatpuffer (0,5 M, pH 9) gegeben. Anschließend werden 4 µl Natriumtartrat (100 mg/ml H₂O), 2 µl Lactose-Lösung (100 mg/ml H₂O) und 1 µl SnCl₂-Lösung (10 mg/ml SnCl₂ x 2 H₂O) zugegeben. Zur Herstellung der SnCl₂-Lösung werden 80 mg/ml in konzentrierter Salzsäure unter kurzem Erhitzen gelöst und mit Wasser auf 10 mg/ml verdünnt. Die benötigte Aktivität von ^{99m}Tc (aus Technetiumgenerator) wird zugegeben und die Lösung anschließend für 30 min auf 95 °C erhitzt, mittels semipräparativer HPLC (siehe 1.3) gereinigt, vom Lösungsmittel befreit und in 300 µl einer sterilen 0.9 %igen NaCI-Lösung aufgenommen.

### 1.5 Herstellung von Liponsäure-y(KKEEE)₃K

Das Peptid y(KKEEE)₃K wird wie unter 1.1 beschrieben mittels Festphasensynthese der Fmoc/*t*Bu-Strategie unter Verwendung der Aminosäuren Fmoc-Lys(Boc)-OH, Fmoc-Glu(O*t*Bu)-OH und Fmoc-Tyr(*t*Bu)-OH (Novabiochem, Merck KGaA, Darmstadt, Deutschland) am Peptidsynthesizer hergestellt. Das Peptid wird zunächst nicht vom Harz abgespalten, sondern nach der finalen Fmoc-Entschützung in NMP suspendiert (pro 100 mg Peptidharz werden 1 ml NMP verwendet). (RS)-Liponsäure (Merck KGaA, Darmstadt, Deutschland; 4 Äquivalente bezogen auf die Harzbeladung) wird währenddessen in NMP (1 ml pro 100 mg) gelöst, mit HBTU (4 Äq.) versetzt und bei Raumtemperatur für ca. 10 min gerührt. Das Reaktionsgemisch wird zum Peptidharz addiert, mit DIPEA (10 Äq.) versetzt und bei Raumtemperatur für ca. 4 h geschüttelt. Das Harz wird 5 x mit NMP und 5 x mit Dichlormethan (DCM) gewaschen und im Vakuum für ca. 4 h getrocknet. Das Liponsäure-Peptid-Konjugat wird mit TFA/Thioanisol /Anisol (90/8/2) für ca. 1 h bei Raumtemperatur vom Harz abgespalten, in kaltem MTBE ausgefällt, mittels Zentrifugation (4000 rpm, 5 min) aufgetrennt, im Vakuum getrocknet, aus Acetonitril/H₂O (1 : 1) lyophilisiert und wie unter 1.2 beschrieben aufgereinigt.

Auf analoge Weise lassen sich auch Konjugate mit anderen Wirkstoffen herstellen.

### 1.6 Herstellung von yKKK(Diacetylkaffeesäure)(EEEKK)₂K(Diacetylkaffeesäure)EEEK

Für die peptische Konjugation von Diacetylkaffeesäure an eine Lysinseitenkette wird die Aminosäure Fmoc-Lys(Mmt)-OH in die Sequenz des Peptidrückgrats eingebaut. Das wie unter 1.1 hergestellte Peptid-Harz wird dabei vor der Abspaltung 3 min mit Dichlormethan (DCM)/Triisopropylsilan/TFA (94 : 5 : 1) versetzt und 5 x mit DCM gewaschen. Dieser Vorgang wird 3 x wiederholt. Für die Kupplung an die orthogonalentschützte Seitenkette von Lysin werden 4 äq der Diacetylkaffeesäure in NMP gelöst, mit 4 äq 1-Ethyl-3-(3-dimethylaminopropyl)-carbodiimid (EDC), 4 äq Ethylcyano(hydroxyimino)acetate (Oxyma Pure) und 10 äq Diisopropylethylamin (DIPEA) versetzt, bei Raumtemperatur für ca. 10 min gerührt und anschließend zum Peptid-Harz addiert. Das Reaktionsgemisch wird für ca. 1 h bei Raumtemperatur geschüttelt, 5 x mit NMP und 5 x mit DCM gewaschen und im Vakuum getrocknet. Das funktionalisierte Peptid wird wie unter 1.1 beschrieben vom Harz abgespalten und wie unter 1.2 beschrieben aufgereinigt.

Auf analoge Weise lassen sich auch Konjugate mit anderen Wirkstoffen herstellen.

### 1.7 Herstellung von y(KKKε(Liponsäure)EEE)₃K

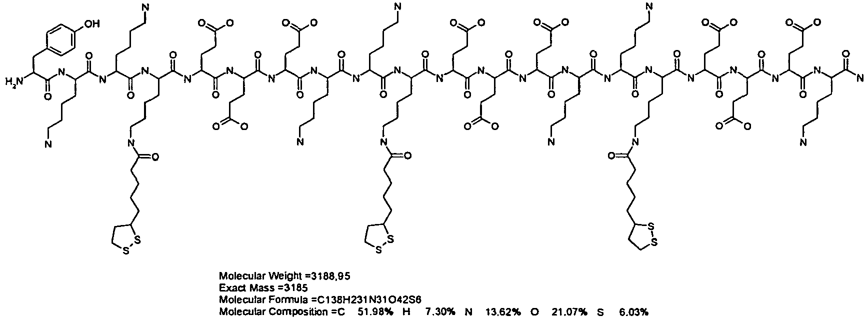

### 1.7.1 Synthese des Fmoc-Lysin(ε-Liponsäure)-OH-Bausteins

N-Hydroxysuccinimid (1,15 g, 10 mmol), α-Liponsäure (2,02 g, 9,8 mmol) und (1,92 g, 10 mmol) 1-Ethyl-3-(3-dimethylaminopropyl)carbodiimid (EDAC) werden in 50 ml DMF gelöst und bei Raumtemperatur für ca. 4 h gerührt. Danach wird der Ansatz mit 60 ml Ethylacetat versetzt. Die organische Phase wird dreimal mit 60 ml destilliertem Wasser, dreimal mit 60 ml gesättigter Natriumhydrogencarbonat-Lösung und einmal mit gesättigter Natriumchlorid-Lösung gewaschen. Die Ethylacetat-Phase wird über Na₂SO₄ getrocknet, filtriert und bis zur Trockene eingeengt.
Ausbeute: 2,23 g (73,5 %)

Fmoc-Lys-OH (2,65 g, 7,2 mmol) wird in 110 ml HEPES-Puffer (pH= 7,4) suspendiert und mit (2,14 g, 7,05 mmol) Liponsäureaktivester (gelöst in 130 ml Acteon) versetzt und bei Raumtemperatur gerührt. Nach ca. 3 h Reaktionszeit wird die Lösung mittels 0,1 N NaOH-Lösung auf pH 7 eingestellt und bei Raumtemperatur für ca. 20 h gerührt. Danach wird der Ansatz mit 0.1 N NaOH auf pH 9 gebracht und zweimal mit ca. 30 ml Ethylacetat gewaschen, anschließend wird mit 1 N HCl auf pH 3 gestellt und dreimal mit ca. 40 ml Ethylacetat extrahiert. Die vereinigten org. Phasen werden mit gesättigter Natriumchlorid-Lösung gewaschen, über Na2SO4 getrocknet, filtriert und bis zur Trockene eingeengt.
Auswaage Rohprodukt: 4,14 g (103, 25 %)

Die Aufreinigung des Rohproduktes erfolgt über Flash-Chromatographie (stationäre Phase: Kieselgel 60, Korngröße: 15 - 40 µm, vorgepackt von der Firma Götec-Labortechnik GmbH, mobile Phase: Chloroform, Methanol (mit 0,1% HOAc), Fluss: 60 ml/min, Beladung: ca. 2 g, Gradient: von 100 % auf 75 % Chloroform in 18 min). Die Produktfraktionen (Rt = 9,1 min) werden vereinigt und bis zur Trockene eingeengt.
Auswaage: 3,18 g (77 %)

### 1.7.2 Festphasen-Peptidsynthese

Peptide werden mit einem Synthesizer der Firma Applied Biosystems GmbH (Carlsbad, CA, USA), Modell 433A, unter Verwendung der Fmoc/tBu-Strategie hergestellt. Die reaktiven Seitenketten der Aminosäuren werden wie folgt geschützt: Lys(Boc), Glu(tBu) und Tyr(tBu). Rink-Amid-Harz der Firma Rapp-Polymere GmbH (Beladungsgrad: 0,24 mmol/g) dient als feste Phase. Die entsprechenden Aminosäuren, der Fmoc-Lysin(ε-Liponsäure)-OH-Baustein und HBTU werden in 4-fachem Überschuss eingesetzt. Als Lösungsmittel wird NMP verwendet und zur jeweiligen Fmoc-Abspaltung Piperidin benutzt (20% in NMP).

Das geschützte Peptid wird mit TFA:Thioanisol:Anisol = 90:8:2 (1 ml pro 100 mg) vom Harz abgespalten (1 - 2 h), in MTBE ausgefällt, zentrifugiert und getrocknet.

### 1.7.3 Radioaktive Iodierung von Peptiden

Die Tyrosin-haltigen Peptide werden mit ¹²⁵Iod mittels der Chloramin-T-Methode markiert. Zur Markierung wirde eine 1 mM Stammlösung in Wasser verwendet. Wenn nötig, wird DMSO zur besseren Löslichkeit zugesetzt. Dazu wird 10 µl der Stammlösung mit 20 µl Phosphatpuffer (0,25 M, pH 7,4) versetzt und die gewünschte Menge an radioaktivem Iod zugegeben. Zur Markierung werden 5 µl Chloramin-T (2 mg/ml H₂O) hinzugefügt. Die Reaktion erfolgt für 30 Sekunden und wird anschließend mit 10 µl einer gesättigten Methionin-Lösung abgebrochen.

Nach der Markierung wird das Peptid mittels semi-präparativer HPLC aufgereinigt, um das überschüssige freie Iod und andere Nebenprodukte zu entfernen. 100 µl der 0,1 mM Stammlösung werden jeweils für die Injektion verwendet. Vor der Injektion wird die Radioaktivität durch einen Geigerzähler erfasst.

Auf analoge Weise lassen sich auch Konjugate mit anderen Wirkstoffen herstellen.

### 2. Anwendungsbeispiele

### 2.1. Organverteilungsstudien

Zur Ermittlung der Pharmakokinetik werden die zu untersuchenden radioaktiv markierten Moleküle weiblichen NMRI Mäusen über die Schwanzvene injiziert (etwa 100 µl pro Tier). Anschließend werden die Tiere (n = 3 pro Zeitpunkt) zu den entsprechenden Zeitpunkten getötet, seziert und die Verteilung der Radioaktivität in den isolierten Organen (Leber, Niere, Lunge, Milz, Darm, Gehirn, Herz, Blut, ...) mittels γ-Counter (Berthold LB951G) quantifiziert. Die gemessene Radioaktivität pro Gramm Organ/Gewebe bezogen auf die injizierte Dosis (ID) wird bestimmt und als %ID/g angegeben.

### 2.2. Einfluss der Kettenlänge und der Verknüpfungsstelle des Wirkstoffs

In weiteren Versuchen wird der Molekülaufbau verändert.

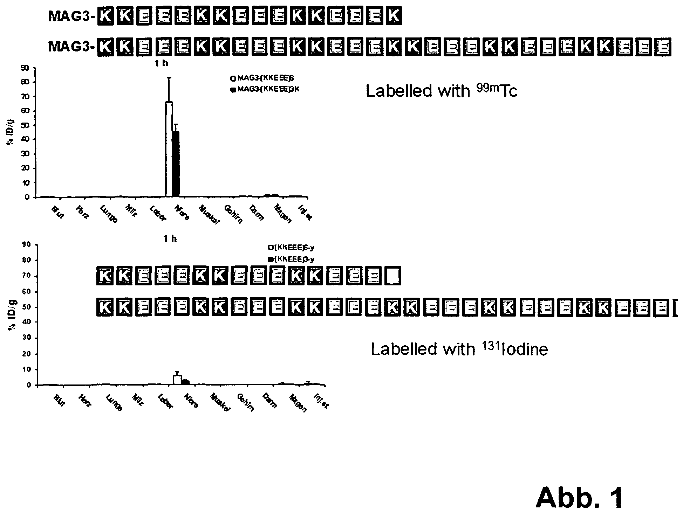

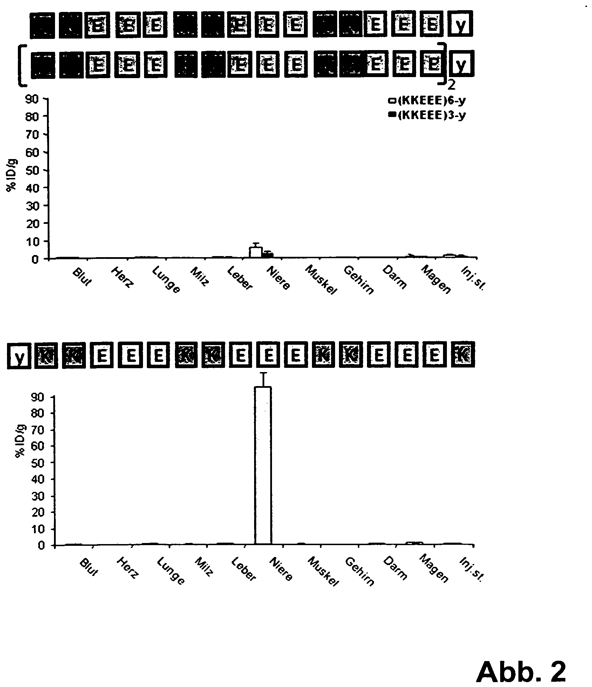

Untersucht werden die Strukturen MAG3-KKEEEKKEEEKKEEEK, MAG3-KKEEEKKEEEKKEEEKKEEEKKEEEKKEEE und y-KKEEEKKEEEKKEEEK (N-terminale Verknüpfung des Wirkstoffs-Abbildung 1, oben und Abbildung 2 unten) und die Strukturen KKEEEKKEEEKKEEE-y und KKEEEKKEEEKKEEEKKEEEKKEEEKKEEE-y (C-terminale Verknüpfung des Wirkstoffs - Abbildung 1, unten und Abbildung 2, oben)
y steht hierbei für D-Tyrosin; MAG3 für ein Peptidfragment, welches ^{99m}Tc komplexiert.

Das Ergebnis ist in Abbildung 1 und 2 dargestellt (ID/g steht hierbei für "Injected Dose per Gram of Tissue): Die Freisetzung von radiomarkiertem Tyrosin (als "Wirkstoff") wird zum einen über die Kettenlänge und zum anderen über die Verknüpfungsstelle des "Wirkstoffes" (C- oder N-Terminus) stark beeinflusst. Grundsätzlich führen längere Peptide zu einer verzögerten Freisetzung. Außerdem verläuft die Freisetzung C-terminal-verknüpfter Tracer (lod-Tyrosin oder auch MAG3 mit ^{99m}Tc) wesentlich schneller als bei N-terminaler Verknüpfung. Der geschwindigkeitsbestimmende Schritt beim Peptidabbau wird offensichtlich vor allem durch Carboxypeptidasen beeinflusst, die vom C-Terminus aus beginnend das Peptid abbauen.
Durch den molekularen Aufbau des Peptides und den Ort der Verknüpfung des Wirkstoffes (C- oder N-Terminus) kann die Freisetzungskinetik eines Wirkstoffes bewusst eingestellt werden.

### 2.3. Einfluss des Verzweigungsgrades

Der enzymatische Abbau verzweigter Peptidstrukturen ist grundsätzlich deutlich schwieriger als der Abbau linearer Peptide. Hierzu wird ein vergleichender Versuch durchgeführt, bei dem der Modellwirkstoff Radiolod-Tyrosin linear in die Kette oder verzweigt in die Kette eingebaut wurde. Untersucht werden die Strukturen KKEEEKK(y)EEEKKEEE und KKEEEKyEEEKKEEEK.

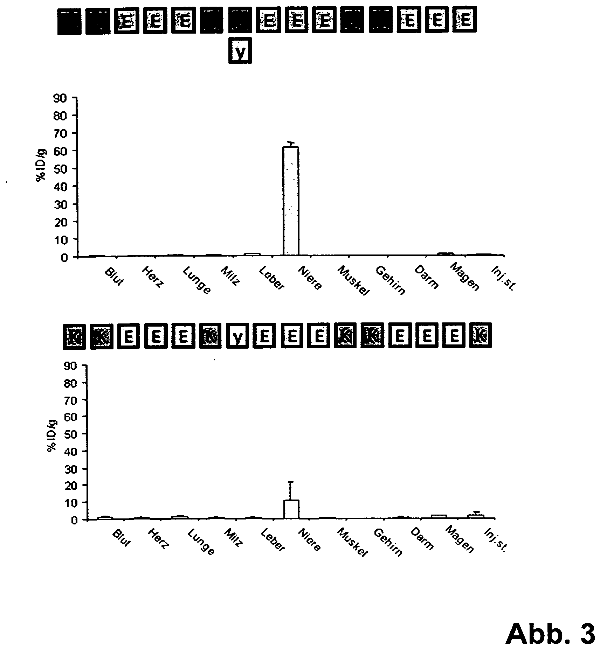

Das Ergebnis wird in Abbildung 3 dargestellt: Die Abbildung zeigt die Organverteilung eine Stunde nach Verabreichung. Das verzweigt in der Kette eingebaute Radio-Iod-Tyrosin (Abbildung 3, oben) wird dabei deutlich langsamer als das lineare abgebaut (Abbildung 3, unten).

### 2.4. Einfluss der Verknüpfungsart

Untersucht werden die Strukturen y-KKEEEKKEEEKKEEEK (Verknüpfung des Wirkstoffs über Amidbindung) und yoKKEEEKKEEEKKEEEK (Verknüpfung des Wirkstoffs über Esterbindung).

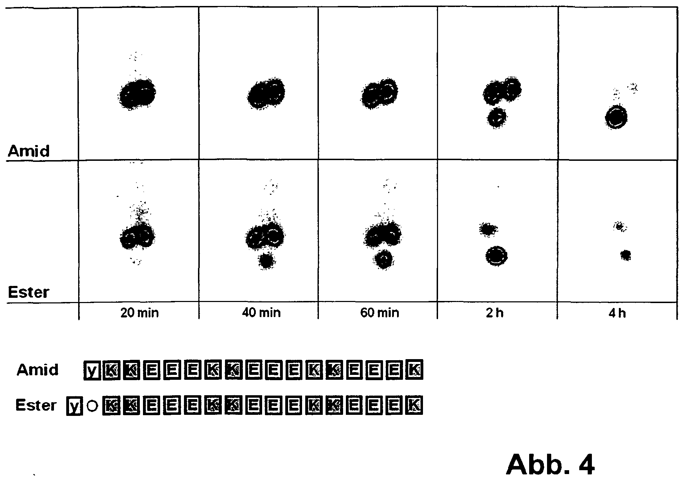

Das Ergebnis ist in Abbildung 4 gezeigt: Für die Anbindung von Wirkstoffen, die schnell freigesetzt werden sollen, hat sich der Einbau einer leicht spaltbaren Esterverknüpfung des Wirkstoffes mit dem Wirkstofftransporter als vorteilhaft erwiesen (Abbildung 4, unten). Die Auftrennung der Esterverknüpfung erfolgt zeitlich schneller als der Abbau des Wirkstofftransporters durch Proteasen.

### 2.5. Vergleichsversuche

Um die Nierenspezifität der in der Literatur genannten Peptide mit den erfindungsgemäßen Strukturen vergleichen zu können, werden die in der Literatur bekannten Peptide APASLYN und HITSLLS (Denby et al.: Molecular Therapy 15, 9, 2007, 1647-1654) mithilfe eines Peptidsynthesizers der Firma Applied Bioscience, Modell 433A, hergestellt und mit einem realen Wirkstoff konjugiert. Die Peptidsequenzen werden mittels Fmoc-Strategie aufgebaut und der Wirkstoff durch Festphasenreaktion an den Carrier konjugiert. Als weiterer Vergleich wird eine beliebige Peptidsequenz y(MARIA)₃ mit 16 Aminosäuren ausgewählt. In den Fällen, in denen kein Tyrosin zur Radiomarkierung im Peptid vorhanden ist (HITSLLS) wird zusätzlich ein D-Tyrosin eingefügt. Als Wirkstoff wird (*RS*)-Liponsäure, (Abk.: LA) verwendet.

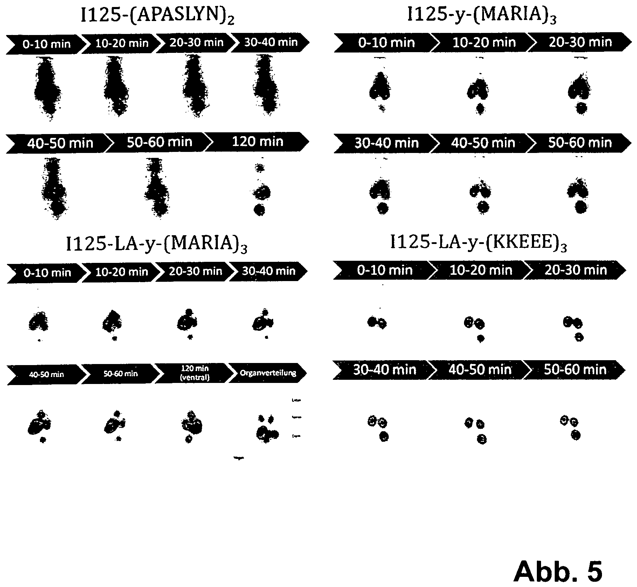

Abbildung 5 zeigt die SPECT-Aufnahmen der Verteilung der Peptide (APASLYN)₂ und y(MARIA)₃ im Tiermodell Maus nach verschiedenen Zeiten. Im Beispiel von (APASLYN)₂ ist die Nierenselektivität des Peptids bereits ohne Wirkstoff Liponsäure unzureichend. Die beliebig ausgewählte Peptidsequenz y(MARIA)₃ weist zwar eine gute Nierenselektivität auf, die aber nach Konjugation mit Liponsäure weitgehend verloren geht. Im Vergleich dazu bleibt die Nierenselektivität bei der erfindungsgemäßen Struktur (KKEEE)₃ auch mit konjugierter Liponsäure erhalten.

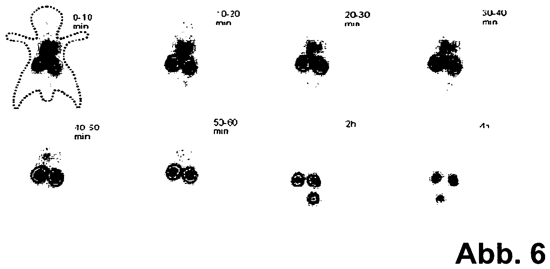

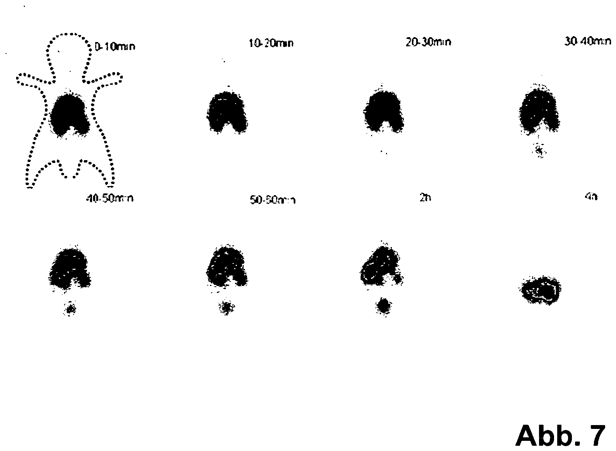

In einem weiteren Versuch wird die Glutaminsäure im Peptid y(KKEEE)₃K-NH₂ durch Glutamin ersetzt. Das dabei erhaltene Peptid y(KKQQQ)₃K-NH₂ enthält keine Säuregruppen. Untersucht wird die Aufnahme des Peptids an sich und des mit Liponsäure konjugierten Peptids.
Ergebnis (siehe Abbildung 6 und 7): Nach der Markierung des Peptids mit Radioiod lässt sich eine hohe Aufnahme des Peptids in den Nieren mittels Bildgebung nachweisen (Abbildung 6). Nach der Konjugation des Peptids mit dem Wirkstoff Liponsäure geht diese Nierenspezifität jedoch nahezu vollständig verloren (Abbildung 7).

In einem weiteren Versuch wird ein Peptid synthetisiert, das nur aus säuregruppentragenden Aminosäuren aufgebaut ist (yD₈). Dieses Peptid wird ebenfalls mit Radioiod markiert und NMRI-Mäusen intravenös appliziert.

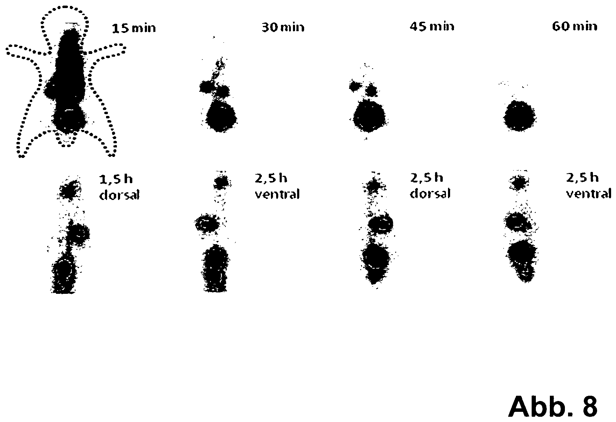

Abbildung 8 zeigt das Ergebnis: Bereits nach 15 Minuten befindet sich der größte Teil der verabreichten Radioaktivität in der Harnblase der Versuchstiere. Ein vollständig aus sauren Aminosäuren aufgebautes Peptid wird offensichtlich sehr schnell über die Nieren ausgeschieden und nicht von den proximalen Tubuluszellen der Nieren rückresorbiert. Eine solcher, rein saurer Molekülaufbau ist für den Transport von Wirkstoffen in die proximalen Tubuluszellen offensichtlich nicht geeignet.

### 2.6. Szintigraphische Verteilungen von y(KKEE)₅K, y(KKKEE)₃K und y(RREEE)₃R

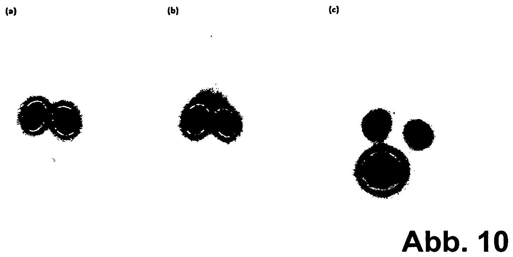

In weiteren Versuchen wird das Säure-Basen-Verhältnis variiert und Lysin gegen Arginin ausgetauscht. Folgende Peptide werden dabei untersucht: y(KKEE)₅K, y(KKKEE)₃K und y(RREEE)₃R.
Ergebnis (siehe Abbildung 10, 1 h-Werte nach jeweiliger radioaktiver Markierung der Peptide mit lod-125 und szintigraphischer Untersuchung nach intravenöser Applikation in weiblichen NMRI-Mäusen): Die Veränderung des Säure-Basen-Verhältnisses von 1 : 1 (y(KKEE)₅K) (Abbildung 10(a)) auf 2 : 3 (y(KKKEE)₃K) (Abbildung 10(b)) verändert die Nierenselektivität der Peptide nicht nachweislich. Beim Austausch des Lysins gegen Arginin (y(RREEE)₃R) befindet sich allerdings nach 1 h der größte Teil der verabreichten Radioaktivität in der Harnblase der Versuchstiere. Die hohe Nierenspezifität bleibt erhalten, kein anderes Organ wird adressiert, allerdings scheint die renale Eliminierung durch den Austausch der Aminosäure beschleunigt zu werden (siehe Abbildung 10(c)).

### 2.7. Szintigraphische Verteilung von Diacetylkaffeesäure-Konjugaten

In weiteren Versuchen wird der potentielle Wirkstoff Diacetylkaffeesäure (DCA) sowohl N-terminal als auch mehrfach an Lysinseitenketten des Peptidrückgrats angebunden. Die Herstellung des N-terminalen Konjugats mit y(KKEEE)₃K erfolgt analog wie unter 1.5. beschrieben; die Herstellung des zweifach konjugierten Moleküls (Struktur: yKKK(DCA)EEEKKEEEKKK(DCA)EEEK) wie unter 1.6. beschrieben. Die so erhaltenen Peptid-Wirkstoff-Konjugate werden nach der Markierung mittels lod-125 und intravenöser Applikation im Tiermodell Maus auf ihre Nierenselektivität untersucht.

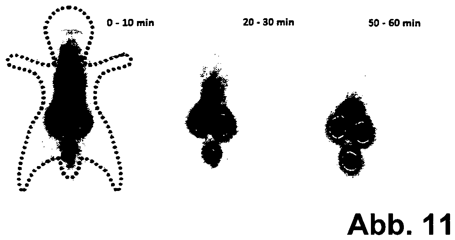

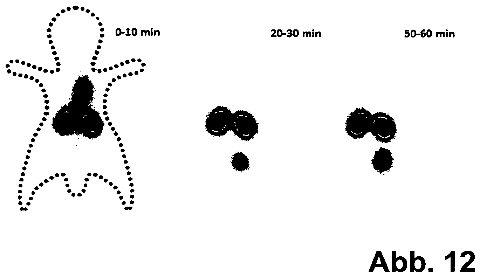

Ergebnis (siehe Abbildung 11 und 12): Die hergestellten Peptid-Wirkstoffkonjugate behalten sowohl nach der N-terminalen Anbindung von Diacetylkaffeesäure (Abbildung 11) als auch bei 2facher Anbindung von Diacetylkaffeesäure an unterschiedliche Seitenketten von Lysin des Peptidrückgrats (Abbildung 12) ihre hohe Nierenspezifität bei.

### 2.8. Administrationsroute

In weiteren Versuchen wird die Administrationsroute untersucht.

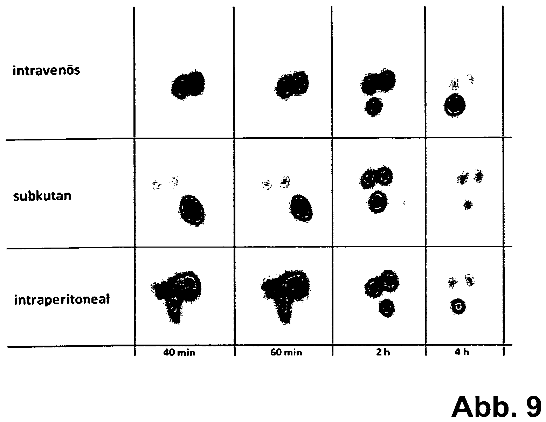

Dazu werden neun NMRI-Mäuse in drei Gruppen eingeteilt. Alle Tiere erhalten 10 mg/kg KG eines Konjugates von D-Tyrosin N-terminal an (KKEEE)₃K gebunden. Ein Teil des Konjugates wird mittels Chloramin-T-Methode am D-Tyrosin mit einem radioaktivem lodisotop markiert. Gruppe 1 wird das markierte Konjugat intravenös, Gruppe 2 subkutan und Gruppe 3 intraperitoneal verabreicht. Das Konjugat ist dabei in 100 µl PBS-Puffer gelöst. Zu verschiedenen Zeiten (40, 60, 120 und 240 Minuten) werden nun SPECT-Aufnahmen von Tieren aus der jeweiligen Gruppe erstellt. Die Ergebnisse dieser Versuchsreihe sind in Abbildung 9 dargestellt.

Neben der in der Literatur beschriebenen intravenösen Gabe von Peptiden/Proteinen zum Wirkstofftransport in die Nieren kann auch die subkutane und intraperitoneale Gabe der erfindungsgemäßen Peptide bzw. Peptid-Wirkstoff-Konjugate erfolgreich die Nieren adressieren.

### 2.9 Szintigraphische Verteilung von Liponsäure-Konjugaten nach Beispiel 1.7

In weiteren Versuchen wird der potentielle Wirkstoff Liponsäure über die Lysinseitenketten des Peptidrückgrats angebunden. Die Herstellung des Konjugats y(KKKε(Liponsäure)EEE)₃K erfolgt wie in Beispiel 1.7. beschrieben. Das so erhaltene Peptid-Wirkstoff-Konjugat wird nach der Markierung mittels lod-125 und intravenöser Applikation im Tiermodell Maus auf seine Nierenselektivität untersucht.

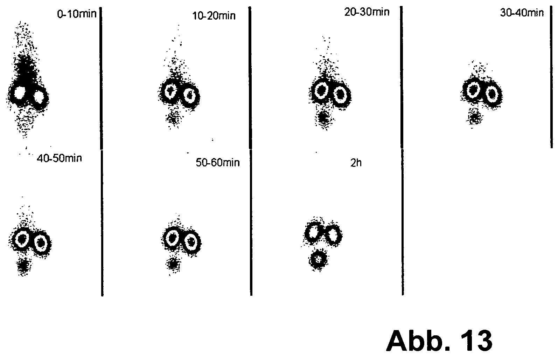

Ergebnis (siehe Abbildung 13): Das hergestellte Peptid-Wirkstoffkonjugat weist eine hohe Nierenspezifität auf.

## Patentansprüche

1. Peptid, welches zu mehr als 50 % (bezogen auf die Anzahl der Aminosäureeinheiten) aus Sequenzabschnitten ausgewählt aus der Gruppe umfassend -(KKEEE)-, -(RREEE)-, -(KKEE)-, -(KKKEEE)- und -(KKKEE)- besteht,
und wobei
- das Peptid zu mindestens 80 % (bezogen auf die Anzahl der Aminosäureeinheiten) aus den Aminosäuren K und E bzw. R und E besteht,
und das Peptid 3 bis 5 Sequenzabschnitte wie zuvor definiert enthält, oder
Konjugat enthaltend mindestens ein Peptid wie zuvor definiert und mindestens einen kovalent, optional über einen Spacer, gebundenen Wirkstoff,
zur Verwendung zum Targeting der Niere.

2. Peptid welches zu mehr als 50 % (bezogen auf die Anzahl der Aminosäureeinheiten) aus Sequenzabschnitten ausgewählt aus der Gruppe umfassend -(KKEEE)-, -(RREEE)-, -(KKEE)-, -(KKKEEE)- und -(KKKEE)- besteht,
und wobei
- das Peptid zu mindestens 80 % (bezogen auf die Anzahl der Aminosäureeinheiten) aus den Aminosäuren K und E bzw. R und E besteht,
und das Peptid 3 bis 5 Sequenzabschnitte wie zuvor definiert enthält, zur Verwendung zum Schutz der Niere.

3. Peptid oder Konjugat zur Verwendung nach Anspruch 1 oder 2, **dadurch gekennzeichnet, dass** das Peptid für ein Peptid ausgewählt aus der Gruppe umfassend (RREEE)₃R, (KKEE)₅K, (KKKEE)₃K, (KKKEEE)₃K und (KKEEE)₃K steht.

4. Konjugat zur Verwendung nach Anspruch 1 oder 3, **dadurch gekennzeichnet, dass** der Wirkstoff ausgewählt ist aus der Gruppe der **Immunsuppressiva** z. B. Azathioprin, Mycophenolat-Mofetil, Ciclosporin, Tacrolimus, Sirolimus, Fingolimod oder Triptolid, **Zytostatika** z. B. Atrasentan, Nintedanib, Bleomycin, Dactinomycin, Mitomycin, Daunorubicin, Doxorubicin, Epirubicin, Idarubicin, Mitoxantron, Amsacrin, Doxofluridin, Cisplatin, Carboplatin, Oxaliplatin, Satraplatin, Camptothecin, Toptecan, Irinotecan, Etoposid, Teniposid, Cyclophosphamid, Trofosfamid, Melphalan, Chlorambucil, Estramustin, Busulfan, Chlorambucil, Chlormethin, Treosulfan, Carmustin, Lomustin, Nimustin, Procarbazin, Streptozocin, Dacarbazin, Ifosfamid, Temozolomid, Thiotepa, Vinorelbin, Vincristin, Vinblastin, Vindesin, Paclitaxel, Docetaxel, Methotrexat, Pemetrexed, Raltitrexed, Fluorouracil, Capecitabin, Cytosinarabinosid, Gemcitabin, Tioguanin, Pentostatin, Mercaptopurin, Fludarabin, Caldribin, Hydroxycarbamid, Mitotan, Azacitidin, Cytarabin, Nelarabin, Bortezomib, Anagrelid insbesondere der Protein-Kinase-Inhibitoren wie z. B. Imatinib, Erlotinib, Sunitinib, Sorafenib, Dasatinib, Lapatinib oder Nilotinib, **Immuntherapeutika** z. B. Cetuximab, Alemtuzumab und Bevacizumab, **Antiphlogistika** z. B. Naproxen, Ibuprofen, Indometacin, Prednisolon, Prednison, Hydrocortison oder Budesonid, **Antibiotika** insbesondere der Penicilline wie z. B. Benzylpenicillin, Methicillin oder Amoxicillin, der Cephalosporine wie z. B. Cefuroxim, Cefotaxim, Cefadroxil oder Cefixim, der β-Lactamase-Inhibitoren wie z. B. Clavulansäure, Sulbactam oder Tazobactam, der Carbapeneme wie z. B. Imipenem oder Meropenem, der Monobactame wie z. B. Aztreonam, der Tetracycline wie z. B. Tetracyclin, Chlortetracyclin, Oxytetracyclin, Doxycyclin, Minocyclin oder Tigecyclin, der Makrolid-Antibiotika wie z. B. Erythromycin A, der Glykopeptid-Antibiotika wie z. B. Vancomycin, der Endiine wie z.B. Calicheamicin, **Virostatika** z. B. Aciclovir, Valaciclovir, Ganciclovir, Valganciclovir, Penciclovir, Famciclovir, Brivudin, Cidofovir, Foscarnet, Idoxuridin oder Tromantadin, **Antihypertensiva** insbesondere der **ACE-Hemmer** wie z. B. Benazepril, Captopril, Cilazapril, Enalapril, Fosinopril, Lisinopril, Perindopril, Quinapril, Ramipril, Trandolapril oder Zofenopril, der **Sartane** wie z. B. Losartan, Balsartan, Irbesartan, Candesartan, Eprosartan, Olmesartan oder Telmisartan, der **Renin-Inhibitoren** wie z. B. Aliskiren und der **Betablocker** wie z. B. Proproanolol, Pindolol, Sotalol, Bopindolol, Atenolol, Bisorpolol, Celiprolol, Esmolol, Metoprolol, Nebivolol, Oxprenolol, Carvedilol oder Labetalol, **Urikosurika** z. B. Probenecid oder Benzbromaron oder **Diuretika** z. B. Acetazolamid, Furosemid, Torasemid, Bumetanid, Piretanid, Azosemid, Etacrynsäure, Etozolin, Hydrochlorothiazid, Benzthiazid, Chlorothiazid, Chlorthalidon, Indapamid, Mefrusid, Metolazon, Clopamid, Xipamid, Hydroflumethiazid, Methyclothiazid, Polythiazid, Amilorid, Triameteren, Spironolacton, Canrenon, Eplerenon oder Spironolacton, **Antifibrotika** z. B. Pirfenidon oder Seliciclib.

5. Konjugat zur Verwendung nach einem oder mehreren der Ansprüche 1 und 3 bis 4, **dadurch gekennzeichnet, dass** der mindestens eine Wirkstoff an den N-Terminus und/oder den C-Terminus des Peptids gebunden ist.

6. Konjugat zur Verwendung nach einem oder mehreren der Ansprüche 1 und 3 bis 4, **dadurch gekennzeichnet, dass** der mindestens eine Wirkstoff an eine Aminosäure innerhalb der Kette gebunden ist.

7. Konjugat zur Verwendung nach einem oder mehreren der Ansprüche 1 und 3 bis 6, **dadurch gekennzeichnet, dass** der Wirkstoff über eine Esterverknüpfung gebunden ist.

8. Peptid ausgewählt aus der Gruppe umfassend (RREEE)₃R, (KKEE)₅K, (KKKEE)₃K, (KKKEEE)₃K und (KKEEE)₃K.

9. Konjugat enthaltend mindestens ein Peptid, welches zu mehr als 50 % (bezogen auf die Anzahl der Aminosäureeinheiten) aus Sequenzabschnitten ausgewählt aus der Gruppe umfassend - (KKEEE)-, -(RREEE)-, -(KKEE)-, -(KKKEEE)- und -(KKKEE)- steht und wobei
- das Peptid zu mindestens 80 % (bezogen auf die Anzahl der Aminosäureeinheiten) aus den Aminosäuren K und E bzw. R und E besteht,
- und das Peptid 3 bis 5 aufeinanderfolgende Sequenzabschnitte wie zuvor definiert enthält
und mindestens einen kovalent, optional über einen Spacer, gebundenen Wirkstoff, wobei der Wirkstoff ausgewählt ist aus der Gruppe der **Immunsuppressiva** z. B. Azathioprin, Mycophenolat-Mofetil, Ciclosporin, Tacrolimus, Sirolimus, Fingolimod oder Triptolid, **Zytostatika** z. B. Atrasentan, Nintedanib, Bleomycin, Dactinomycin, Mitomycin, Daunorubicin, Doxorubicin, Epirubicin, Idarubicin, Mitoxantron, Amsacrin, Doxofluridin, Cisplatin, Carboplatin, Oxaliplatin, Satraplatin, Camptothecin, Toptecan, Irinotecan, Etoposid, Teniposid, Cyclophosphamid, Trofosfamid, Melphalan, Chlorambucil, Estramustin, Busulfan, Chlorambucil, Chlormethin, Treosulfan, Carmustin, Lomustin, Nimustin, Procarbazin, Streptozocin, Dacarbazin, Ifosfamid, Temozolomid, Thiotepa, Vinorelbin, Vincristin, Vinblastin, Vindesin, Paclitaxel, Docetaxel, Methotrexat, Pemetrexed, Raltitrexed, Fluorouracil, Capecitabin, Cytosinarabinosid, Gemcitabin, Tioguanin, Pentostatin, Mercaptopurin, Fludarabin, Caldribin, Hydroxycarbamid, Mitotan, Azacitidin, Cytarabin, Nelarabin, Bortezomib, Anagrelid insbesondere der Protein-Kinase-Inhibitoren wie z. B. Imatinib, Erlotinib, Sunitinib, Sorafenib, Dasatinib, Lapatinib oder Nilotinib, **Immuntherapeutika** z. B. Cetuximab, Alemtuzumab und Bevacizumab, **Antiphlogistika** z. B. Naproxen, Ibuprofen, Indometacin, Prednisolon, Prednison, Hydrocortison oder Budesonid, **Antibiotika** insbesondere der Penicilline wie z. B. Benzylpenicillin, Methicillin oder Amoxicillin, der Cephalosporine wie z. B. Cefuroxim, Cefotaxim, Cefadroxil oder Cefixim, der β-Lactamase-Inhibitoren wie z. B. Clavulansäure, Sulbactam oder Tazobactam, der Carbapeneme wie z. B. Imipenem oder Meropenem, der Monobactame wie z. B. Aztreonam, der Tetracycline wie z. B. Tetracyclin, Chlortetracyclin, Oxytetracyclin, Doxycyclin, Minocyclin oder Tigecyclin, der Makrolid-Antibiotika wie z. B. Erythromycin A, der Glykopeptid-Antibiotika wie z. B. Vancomycin, der Endiine wie z.B. Calicheamicin, **Virostatika** z. B. Aciclovir, Valaciclovir, Ganciclovir, Valganciclovir, Penciclovir, Famciclovir, Brivudin, Cidofovir, Foscarnet, Idoxuridin oder Tromantadin, **Antihypertensiva** insbesondere der **ACE-Hemmer** wie z. B. Benazepril, Captopril, Cilazapril, Enalapril, Fosinopril, Lisinopril, Perindopril, Quinapril, Ramipril, Trandolapril oder Zofenopril, der **Sartane** wie z. B. Losartan, Balsartan, Irbesartan, Candesartan, Eprosartan, Olmesartan oder Telmisartan, der **Renin-Inhibitoren** wie z. B. Aliskiren und der **Betablocker** wie z. B. Proproanolol, Pindolol, Sotalol, Bopindolol, Atenolol, Bisorpolol, Celiprolol, Esmolol, Metoprolol, Nebivolol, Oxprenolol, Carvedilol oder Labetalol, **Urikosurika** z. B. Probenecid oder Benzbromaron oder **Diuretika** z. B. Acetazolamid, Furosemid, Torasemid, Bumetanid, Piretanid, Azosemid, Etacrynsäure, Etozolin, Hydrochlorothiazid, Benzthiazid, Chlorothiazid, Chlorthalidon, Indapamid, Mefrusid, Metolazon, Clopamid, Xipamid, Hydroflumethiazid, Methyclothiazid, Polythiazid, Amilorid, Triameteren, Spironolacton, Canrenon, Eplerenon oder Spironolacton, **Antifibrotika** z. B. Pirfenidon oder Seliciclib.

10. Konjugat nach Anspruch 9, **dadurch gekennzeichnet, dass** der mindestens eine Wirkstoff an den N-Terminus und/oder den C-Terminus des Peptids gebunden ist.

11. Konjugat nach Anspruch 9, **dadurch gekennzeichnet, dass** der mindestens eine Wirkstoff an eine Aminosäure innerhalb der Kette gebunden ist.

12. Konjugat einem oder mehreren der Ansprüche 9 bis 11, **dadurch gekennzeichnet, dass** der Wirkstoff über eine Esterverknüpfung gebunden ist.

13. Verfahren zur Herstellung eines Konjugats nach einem oder mehreren der Ansprüche 9 bis 12, **dadurch gekennzeichnet, dass** ein optional aktivierter Wirkstoff an das Peptid konjugiert wird.

14. Peptid nach Anspruch 8 zur Verwendung als Arzneimittel.

15. Konjugat nach einem oder mehreren der Ansprüche 9 bis 12 zur Verwendung als Arzneimittel.

16. Arzneimittel, insbesondere eine therapeutische oder eine bildverstärkende Zusammensetzung, enthaltend mindestens ein Peptid nach Anspruch 8 oder ein Konjugat nach einem oder mehreren der Ansprüche 9 bis 12.

## Claims

1. Peptide which consists of more than 50% (based on the number of amino acid units) of sequence sections selected from the group comprising -(KKEEE)-, -(RREEE)-, -(KKEE)-, -(KKKEEE)- and -(KKKEE)-, and where
- the peptide consists of at least 80% (based on the number of amino acid units) of amino acids K and E or R and E,
and the peptide contains 3 to 5 sequence sections as defined above, or
conjugate containing at least one peptide as defined above and at least one active compound which is covalently bonded, optionally via a spacer,
for use for targeting of the kidney.

2. Peptide which consists of more than 50% (based on the number of amino acid units) of sequence sections selected from the group comprising -(KKEEE)-, -(RREEE)-, -(KKEE)-, -(KKKEEE)- and -(KKKEE)-, and where
- the peptide consists of at least 80% (based on the number of amino acid units) of amino acids K and E or R and E,
and the peptide contains 3 to 5 sequence sections as defined above, for use for protection of the kidney.

3. Peptide or conjugate for use according to Claim 1 or 2, **characterised in that** the peptide stands for a peptide selected from the group comprising (RREEE)₃R, (KKEE)₅K, (KKKEE)₃K, (KKKEEE)₃K and (KKEEE)₃K.

4. Conjugate for use according to Claim 1 or 3, **characterised in that** the active compound is selected from the group of the **immunosuppressants,** for example azathioprine, mycophenolate-mofetil, ciclosporin, tacrolimus, sirolimus, fingolimod or triptolide, **cytostatics,** for example atrasentan, nintedanib, bleomycin, dactinomycin, mitomycin, daunorubicin, doxorubicin, epirubicin, idarubicin, mitoxantron, amsacrine, doxofluridine, cisplatin, carboplatin, oxaliplatin, satraplatin, camptothecin, toptecan, irinotecan, etoposide, teniposide, cyclophosphamide, trofosfamide, melphalan, chlorambucil, estramustine, busulfan, chlorambucil, chlormethine, treosulfan, carmustine, lomustine, nimustine, procarbazine, streptozocine, dacarbazine, ifosfamide, temozolomide, thiotepa, vinorelbine, vincristine, vinblastine, vindesine, paclitaxel, docetaxel, methotrexate, pemetrexed, raltitrexed, fluorouracil, capecitabine, cytosine arabinoside, gemcitabine, tioguanine, pentostatin, mercaptopurine, fludarabine, caldribine, hydroxycarbamide, mitotane, azacitidine, cytarabine, nelarabine, bortezomib, anagrelide, in particular the protein kinase inhibitors, such as, for example, imatinib, erlotinib, sunitinib, sorafenib, dasatinib, lapatinib or nilotinib, **immunotherapeutic agents**, for example cetuximab, alemtuzumab and bevacizumab, **antiphlogistics**, for example naproxen, ibuprofen, indometacin, prednisolone, prednisone, hydrocortisone or budesonide, **antibiotics**, in particular the penicillins, such as, for example, benzylpenicillin, methicillin or amoxicillin, the cephalosporins, such as, for example, cefuroxim, cefotaxim, cefadroxil or cefixim, the β-lactamase inhibitors, such as, for example, clavulanic acid, sulbactam or tazobactam, the carbapenems, such as, for example, imipenem or meropenem, the monobactams, such as, for example, aztreonam, the tetracyclines, such as, for example, tetracycline, chlortetracycline, oxytetracycline, doxycycline, minocycline or tigecycline, the macrolide antibiotics, such as, for example, erythromycin A, the glycopeptide antibiotics, such as, for example, vancomycin, the enediynes, such as, for example, calicheamicin, **virostatics**, for example aciclovir, valaciclovir, ganciclovir, valganciclovir, penciclovir, famciclovir, brivudine, cidofovir, foscarnet, idoxuridine or tromantadine, **antihypertensives**, in particular the **ACE inhibitors**, such as, for example, benazepril, captopril, cilazapril, enalapril, fosinopril, lisinopril, perindopril, quinapril, ramipril, trandolapril or zofenopril, the **sartans**, such as, for example, losartan, balsartan, irbesartan, candesartan, eprosartan, olmesartan or telmisartan, the **renin inhibitors**, such as, for example, aliskiren, and the **beta blockers**, such as, for example, propranolol, pindolol, sotalol, bopindolol, atenolol, bisorpolol, celiprolol, esmolol, metoprolol, nebivolol, oxprenolol, carvedilol or labetalol, **uricosurics**, for example probenecid or benzbromarone, or **diuretics**, for example acetazolamide, furosemide, torasemide, bumetanide, piretanide, azosemide, etacrynic acid, etozoline, hydrochlorothiazide, benzthiazide, chlorothiazide, chlorthalidone, indapamide, mefruside, metolazone, clopamide, xipamide, hydroflumethiazide, methyclothiazide, polythiazide, amiloride, triameterene, spironolactone, canrenone, eplerenone or spironolactone, **antifibrotics**, for example pirfenidone or seliciclib.

5. Conjugate for use according to one or more of Claims 1 and 3 to 4, **characterised in that** the at least one active compound is bonded to the N terminal and/or the C terminal of the peptide.

6. Conjugate for use according to one or more of Claims 1 and 3 to 4, **characterised in that** the at least one active compound is bonded to an amino acid within the chain.

7. Conjugate for use according to one or more of Claims 1 and 3 to 6, **characterised in that** the active compound is bonded via an ester link.

8. Peptide selected from the group comprising (RREEE)₃R, (KKEE)₅K, (KKKEE)₃K, (KKKEEE)₃K and (KKEEE)₃K.

9. Conjugate containing at least one peptide which consists of more than 50% (based on the number of amino acid units) of sequence sections selected from the group comprising -(KKEEE)-, -(RREEE)-, -(KKEE)-, -(KKKEEE)- and -(KKKEE)-,
and where
- the peptide consists of at least 80% (based on the number of amino acid units) of amino acids K and E or R and E,
- and the peptide contains 3 to 5 successive sequence sections as defined above,
and at least one active compound which is covalently bonded, optionally via a spacer, where the active compound is selected from the group of the **immunosuppressants**, for example azathioprine, mycophenolate-mofetil, ciclosporin, tacrolimus, sirolimus, fingolimod or triptolide, **cytostatics**, for example atrasentan, nintedanib, bleomycin, dactinomycin, mitomycin, daunorubicin, doxorubicin, epirubicin, idarubicin, mitoxantron, amsacrine, doxofluridine, cisplatin, carboplatin, oxaliplatin, satraplatin, camptothecin, toptecan, irinotecan, etoposide, teniposide, cyclophosphamide, trofosfamide, melphalan, chlorambucil, estramustine, busulfan, chlorambucil, chlormethine, treosulfan, carmustine, lomustine, nimustine, procarbazine, streptozocine, dacarbazine, ifosfamide, temozolomide, thiotepa, vinorelbine, vincristine, vinblastine, vindesine, paclitaxel, docetaxel, methotrexate, pemetrexed, raltitrexed, fluorouracil, capecitabine, cytosine arabinoside, gemcitabine, tioguanine, pentostatin, mercaptopurine, fludarabine, caldribine, hydroxycarbamide, mitotane, azacitidine, cytarabine, nelarabine, bortezomib, anagrelide, in particular the protein kinase inhibitors, such as, for example, imatinib, erlotinib, sunitinib, sorafenib, dasatinib, lapatinib or nilotinib, **immunotherapeutic agents**, for example cetuximab, alemtuzumab and bevacizumab, **antiphlogistics**, for example naproxen, ibuprofen, indometacin, prednisolone, prednisone, hydrocortisone or budesonide, **antibiotics**, in particular the penicillins, such as, for example, benzylpenicillin, methicillin or amoxicillin, the cephalosporins, such as, for example, cefuroxim, cefotaxim, cefadroxil or cefixim, the β-lactamase inhibitors, such as, for example, clavulanic acid, sulbactam or tazobactam, the carbapenems, such as, for example, imipenem or meropenem, the monobactams, such as, for example, aztreonam, the tetracyclines, such as, for example, tetracycline, chlortetracycline, oxytetracycline, doxycycline, minocycline or tigecycline, the macrolide antibiotics, such as, for example, erythromycin A, the glycopeptide antibiotics, such as, for example, vancomycin, the enediynes, such as, for example, calicheamicin, **virostatics**, for example aciclovir, valaciclovir, ganciclovir, valganciclovir, penciclovir, famciclovir, brivudine, cidofovir, foscarnet, idoxuridine or tromantadine, **antihypertensives**, in particular the **ACE inhibitors**, such as, for example, benazepril, captopril, cilazapril, enalapril, fosinopril, lisinopril, perindopril, quinapril, ramipril, trandolapril or zofenopril, the **sartans**, such as, for example, losartan, balsartan, irbesartan, candesartan, eprosartan, olmesartan or telmisartan, the **renin inhibitors**, such as, for example, aliskiren, and the **beta blockers**, such as, for example, propranolol, pindolol, sotalol, bopindolol, atenolol, bisorpolol, celiprolol, esmolol, metoprolol, nebivolol, oxprenolol, carvedilol or labetalol, **uricosurics**, for example probenecid or benzbromarone, or **diuretics**, for example acetazolamide, furosemide, torasemide, bumetanide, piretanide, azosemide, etacrynic acid, etozoline, hydrochlorothiazide, benzthiazide, chlorothiazide, chlorthalidone, indapamide, mefruside, metolazone, clopamide, xipamide, hydroflumethiazide, methyclothiazide, polythiazide, amiloride, triameterene, spironolactone, canrenone, eplerenone or spironolactone, **antifibrotics**, for example pirfenidone or seliciclib.

10. Conjugate according to Claim 9, **characterised in that** the at least one active compound is bonded to the N terminal and/or the C terminal of the peptide.

11. Conjugate according to Claim 9, **characterised in that** the at least one active compound is bonded to an amino acid within the chain.

12. Conjugate according to one or more of Claims 9 to 11, **characterised in that** the active compound is bonded via an ester link.

13. Process for the preparation of a conjugate according to one or more of Claims 9 to 12, **characterised in that** an optionally activated active compound is conjugated to the peptide.

14. Peptide according to Claim 8 for use as medicament.

15. Conjugate according to one or more of Claims 9 to 12 for use as medicament.

16. Medicament, in particular a therapeutic or image-enhancing composition, comprising at least one peptide according to Claim 8 or a conjugate according to one or more of Claims 9 to 12.

## Revendications

1. Peptide qui est constitué de plus de 50% (sur la base du nombre d'unités d'acides aminés) de sections de séquences choisies dans le groupe constitué par -(KKEEE)-, -(RREEE)-, -(KKEE)-, -(KKKEEE)- et -(KKKEE)-,
et où
- le peptide est constitué d'au moins 80% (sur la base du nombre d'unités d'acides aminés) d'acides aminés K et E ou R et E,
et le peptide contient de 3 à 5 sections de séquences telles que définies ci-dessus,
ou
conjugué contenant au moins un peptide tel que défini ci-dessus et au moins un composé actif qui est lié de manière covalente, éventuellement via un espaceur,
pour une utilisation dans le ciblage du rein.

2. Peptide qui est constitué de plus de 50% (sur la base du nombre d'unités d'acides aminés) de sections de séquences choisies dans le groupe constitué par -(KKEEE)-, -(RREEE)-, -(KKEE)-, -(KKKEEE)- et -(KKKEE)-,
et où
- le peptide est constitué d'au moins 80% (sur la base du nombre d'unités d'acides aminés) d'acides aminés K et E ou R et E,
et le peptide contient de 3 à 5 sections de séquences telles que définies ci-dessus,
pour une utilisation dans la protection du rein.

3. Peptide ou conjugué pour une utilisation selon la revendication 1 ou 2, **caractérisé en ce que** le peptide représente un peptide choisi dans le groupe constitué par (RREEE)₃R, (KKEE)₅K, (KKKEE)₃K, (KKKEEE)₃K et (KKEEE)₃K.

4. Conjugué pour une utilisation selon la revendication 1 ou 3, **caractérisé en ce que** le composé actif est choisi dans le groupe constitué par les **immunosuppresseurs**, par exemple l'azathioprine, le mycophénolate-mofétil, la ciclosporine, le tacrolimus, le sirolimus, le fingolimod ou le triptolide, les **agents cytostatiques**, par exemple l'atrasentan, le nintédanib, la bléomycine, la dactinomycine, la mitomycine, la daunorubicine, la doxorubicine, l'épirubicine, l'idarubicine, la mitoxantrone, l'amsacrine, la doxifluridine, le cisplatine, le carboplatine, l'oxaliplatine, le satraplatine, la camptothécine, le topotécan, l'irinotécan, l'étoposide, le téniposide, le cyclophosphamide, le trofosfamide, le melphalan, le chlorambucil, l'estramustine, le busulfan, le chlorambucil, la chlorméthine, le tréosulfan, la carmustine, la lomustine, la nimustine, la procarbazine, la streptozocine, la dacarbazine, l'ifosfamide, le témozolomide, le thiotépa, la vinorelbine, la vincristine, la vinblastine, la vindésine, le paclitaxel, le docétaxel, le méthotrexate, le pémétrexed, le raltitrexed, le fluorouracile, la capé-citabine, la cytosine arabinoside, la gemcitabine, la thioguanine, la pentostatine, la mercaptopurine, la fludarabine, la cladribine, l'hydroxycarbamide, le mitotane, l'azacitidine, la cytarabine, la nélarabine, le bortézomib, l'anagrélide, en particulier les inhibiteurs de protéine kinase tels que, par exemple, l'imatinib, l'erlotinib, le sunitinib, le sorafénib, le dasatinib, le lapatinib ou le nilotinib, les **agents immuno-thérapeutiques**, par exemple le cetuximab, l'alemtuzumab et le bevacizumab, les **antiphlogistiques**, par exemple le naproxène, l'ibuprofène, l'indométacine, la prednisolone, la prednisone, l'hydrocortisone ou le budésonide, les **antibiotiques**, en particulier les pénicillines, telles que, par exemple, la benzylpénicilline, la méthicilline ou l'amoxicilline, les céphalosporines, telles que, par exemple, le céfuroxime, le céfotaxime, le céfadroxil ou le céfixime, les inhibiteurs de β-lactamase, tels que, par exemple, l'acide clavulanique, le sulbactam ou le tazobactam, les carbapénèmes, tels que, par exemple, l'imipénem ou le méropénem, les monobactames, tels que, par exemple, l'aztréonam, les tétracyclines, telles que, par exemple, la tétracycline, la chlortétracycline, l'oxytétracycline, la doxycycline, la minocycline ou la tigécycline, les antibiotiques de type macrolide, tels que, par exemple, l'érythromycine A, les antibiotiques de type glycopeptide, tels que, par exemple, la vancomycine, les ènediynes, tels que, par exemple, la calichéamicine, les **virostatiques**, par exemple l'aciclovir, le valaciclovir, le ganciclovir, le valganciclovir, le penciclovir, le famciclovir, la brivudine, le cidofovir, le foscarnet, l'idoxuridine ou la tromantadine, les **antihypertenseurs**, en particulier les **inhibiteurs d'ACE** tels que, par exemple, le bénazépril, le captopril, le cilazapril, l'énalapril, le fosinopril, le lisinopril, le périndopril, le quinapril, le ramipril, le trandolapril ou le zofénopril, les **sartans** tels que, par exemple, le losartan, le valsartan, l'irbésartan, le candésartan, l'éprosartan, l'olmésartan ou le telmisartan, les **inhibiteurs de la rénine** tels que, par exemple, l'aliskiren, et les **bêta-bloquants** tels que, par exemple, le propranolol, le pindolol, le sotalol, le bopindolol, l'aténolol, le bisoprolol, le céliprolol, l'esmolol, le métoprolol, le nébivolol, l'oxprénolol, le carvédilol ou le labétalol, les **uricosuriques,** par exemple le probénécide ou la benzbromarone, ou les **diurétiques**, par exemple l'acétazolamide, le furosémide, le torasémide, le bumétanide, le pirétanide, l'azosémide, l'acide étacrynique, l'étozoline, l'hydrochlorothiazide, le benzthiazide, le chlorothiazide, la chlorthalidone, l'indapamide, le méfruside, la métolazone, le clopamide, le xipamide, l'hydrofluméthiazide, le méthyclothiazide, le polythiazide, l'amiloride, le triamtérène, la spironolactone, la canrénone, l'éplérénone ou la spironolactone, les **agents antifibrotiques**, par exemple la pirfénidone ou le séliciclib.

5. Conjugué pour une utilisation selon l'une ou plusieurs parmi les revendications 1 et 3 à 4, **caractérisé en ce que** le au moins un composé actif est lié à l'extrémité N-terminale et/ou C-terminale du peptide.

6. Conjugué pour une utilisation selon l'une ou plusieurs parmi les revendications 1 et 3 à 4, **caractérisé en ce que** le au moins un composé actif est lié à un acide aminé au sein de la chaîne.

7. Conjugué pour une utilisation selon l'une ou plusieurs parmi les revendications 1 et 3 à 6, **caractérisé en ce que** le composé actif est lié via une liaison ester.

8. Peptide choisi dans le groupe constitué par (RREEE)₃R, (KKEE)₅K, (KKKEE)₃K, (KKKEEE)₃K et (KKEEE)₃K.

9. Conjugué contenant au moins un peptide qui est constitué de plus de 50% (sur la base du nombre d'unités d'acides aminés) de sections de séquences choisies dans le groupe constitué par -(KKEEE)-, -(RREEE)-, -(KKEE)-, -(KKKEEE)- et -(KKKEE)-,
et où
- le peptide est constitué d'au moins 80% (sur la base du nombre d'unités d'acides aminés) d'acides aminés K et E ou R et E,
- et le peptide contient de 3 à 5 sections successives de séquences telles que définies ci-dessus,
et au moins un composé actif qui est lié de manière covalente, éventuellement via un espaceur, où le composé actif est choisi dans le groupe constitué par les **immunosuppresseurs**, par exemple l'azathioprine, le mycophénolate-mofétil, la ciclosporine, le tacrolimus, le sirolimus, le fingolimod ou le triptolide, les **agents cytostatiques**, par exemple l'atrasentan, le nintédanib, la bléomycine, la dactinomycine, la mitomycine, la daunorubicine, la doxorubicine, l'épirubicine, l'idarubicine, la mitoxantrone, l'amsacrine, la doxifluridine, le cisplatine, le carboplatine, l'oxaliplatine, le satraplatine, la camptothécine, le topotécan, l'irinotécan, l'étoposide, le téniposide, le cyclophosphamide, le trofosfamide, le melphalan, le chlorambucil, l'estramustine, le busulfan, le chlorambucil, la chlorméthine, le tréosulfan, la carmustine, la lomustine, la nimustine, la procarbazine, la streptozocine, la dacarbazine, l'ifosfamide, le témozolomide, le thiotépa, la vinorelbine, la vincristine, la vinblastine, la vindésine, le paclitaxel, le docétaxel, le méthotrexate, le pémétrexed, le raltitrexed, le fluorouracile, la capécitabine, la cytosine arabinoside, la gemcitabine, la thioguanine, la pentostatine, la mercaptopurine, la fludarabine, la cladribine, l'hydroxycarbamide, le mitotane, l'azacitidine, la cytarabine, la nélarabine, le bortézomib, l'anagrélide, en particulier les inhibiteurs de protéine kinase tels que, par exemple, l'imatinib, l'erlotinib, le sunitinib, le sorafénib, le dasatinib, le lapatinib ou le nilotinib, les **agents immuno-thérapeutiques,** par exemple le cetuximab, l'alemtuzumab et le bevacizumab, les **antiphlogistiques**, par exemple le naproxène, l'ibuprofène, l'indométacine, la prednisolone, la prednisone, l'hydrocortisone ou le budésonide, les **antibiotiques**, en particulier les pénicillines, telles que, par exemple, la benzylpénicilline, la méthicilline ou l'amoxicilline, les céphalosporines, telles que, par exemple, le céfuroxime, le céfotaxime, le céfadroxil ou le céfixime, les inhibiteurs de β-lactamase, tels que, par exemple, l'acide clavulanique, le sulbactam ou le tazobactam, les carbapénèmes, tels que, par exemple, l'imipénem ou le méropénem, les monobactames, tels que, par exemple, l'aztréonam, les tétracyclines, telles que, par exemple, la tétracycline, la chlortétracycline, l'oxytétracycline, la doxycycline, la minocycline ou la tigécycline, les antibiotiques de type macrolide, tels que, par exemple, l'érythromycine A, les antibiotiques de type glycopeptide, tels que, par exemple, la vancomycine, les ènediynes, tels que, par exemple, la calichéamicine, les **virostatiques**, par exemple l'aciclovir, le valaciclovir, le ganciclovir, le valganciclovir, le penciclovir, le famciclovir, la brivudine, le cidofovir, le foscarnet, l'idoxuridine ou la tromantadine, les **antihypertenseurs**, en particulier les **inhibiteurs d'ACE** tels que, par exemple, le bénazépril, le captopril, le cilazapril, l'énalapril, le fosinopril, le lisinopril, le périndopril, le quinapril, le ramipril, le trandolapril ou le zofénopril, les **sartans** tels que, par exemple, le losartan, le valsartan, l'irbésartan, le candésartan, l'éprosartan, l'olmésartan ou le telmisartan, les **inhibiteurs de la rénine** tels que, par exemple, l'aliskiren, et les **bêta-bloquants** tels que, par exemple, le propranolol, le pindolol, le sotalol, le bopindolol, l'aténolol, le bisoprolol, le céliprolol, l'esmolol, le métoprolol, le nébivolol, l'oxprénolol, le carvédilol ou le labétalol, les **uricosuriques**, par exemple le probénécide ou la benzbromarone, ou les **diurétiques**, par exemple l'acétazolamide, le furosémide, le torasémide, le bumétanide, le pirétanide, l'azosémide, l'acide étacrynique, l'étozoline, l'hydrochlorothiazide, le benzthiazide, le chlorothiazide, la chlorthalidone, l'indapamide, le méfruside, la métolazone, le clopamide, le xipamide, l'hydrofluméthiazide, le méthyclothiazide, le polythiazide, l'amiloride, le triamtérène, la spironolactone, la canrénone, l'éplérénone ou la spironolactone, les **agents antifibrotiques**, par exemple la pirfénidone ou le séliciclib.

10. Conjugué selon la revendication 9, **caractérisé en ce que** le au moins un composé actif est lié à l'extrémité N-terminale et/ou C-terminale du peptide.

11. Conjugué selon la revendication 9, **caractérisé en ce que** le au moins un composé actif est lié à un acide aminé au sein de la chaîne.

12. Conjugué selon l'une ou plusieurs parmi les revendications 9 à 11, **caractérisé en ce que** le composé actif est lié via une liaison ester.

13. Procédé de préparation d'un conjugué selon l'une ou plusieurs parmi les revendications 9 à 12, **caractérisé en ce qu'**un composé actif éventuellement activé est conjugué au peptide.

14. Peptide selon la revendication 8, pour une utilisation comme médicament.

15. Conjugué selon l'une ou plusieurs parmi les revendications 9 à 12, pour une utilisation comme médicament.

16. Médicament, en particulier une composition thérapeutique ou d'amélioration d'image, comprenant au moins un peptide selon la revendication 8 ou un conjugué selon l'une ou plusieurs parmi les revendications 9 à 12.
